# EUROPEAN PATENT APPLICATION

(11) **EP 1 950 206 A1**
(43) Date of publication of application: **30.07.2008**
(21) Application number: 08102734.4
(22) Date of filing: 22.12.2003
(51) Int. Cl.: C07D 267/22, C07D 413/04, A61K 31/395, A61K 31/422, A61P 29/00, C07D 498/04

(54) **Benzoxazocines and their use as monoamine-reuptake inhibitors**

(30) Priority: 20.12.2002 GB 0229743; 18.07.2003 GB 0316914
(62) Divisional of application: 03786172.1
(71) Applicant: Sosei R&D Ltd., Little Chesterford Saffron Walden Essex CB10 1XL (GB)
(72) Inventor: Baxter, Andrew Douglas, Milton Road, Cambridge, Cambridgeshire CB4 0EY (GB)
(74) Representative: Perry, Robert Edward

(57) **Abstract**

A compound of the general formula (1): 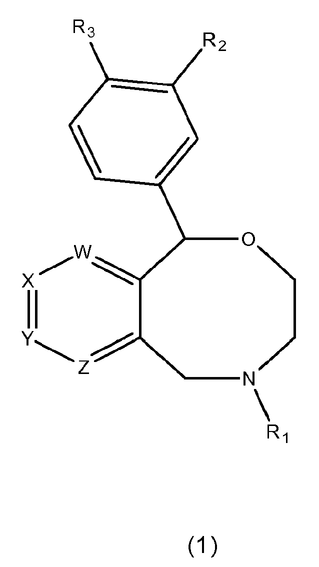 wherein one of W, X, Y and Z is N or CR₄ and the others are each CH;
and R₄ is a specified substituent. These compounds inhibit monoamine reuptake, and are useful in the treatment of pain, emesis depression, post traumatic stress disorders, attention deficit disorders, obsessive compulsive disorders, pre-menstrual syndrome, substance abuse and sexual dysfunction.

## Description

This invention relates to novel benzoxazocine compounds which inhibit monoamine reuptake and their therapeutic use.

### Background of the Invention

Nefopam [(±)-3,4,5,6-tetrahydro-5-methyl-1-phenyl-1H-2,5-benzoxazocine hydrochloride] is a centrally acting non-narcotic analgesic not structurally related to other analgesics. Nefopam has been shown to induce antinociception in animal models of pain and in humans (reviewed in Heel *et al.,* 1980). However, nefopam is not active in the mouse tail-flick test, or the hot plate test and the Randall-Selitto pressure test in rats (Conway and Mitchell, 1977) suggesting that its analgesic mechanism is not opiate-like or anti-inflammatory in nature. Nefopam's antinociception is not blocked by nalaxone further suggesting that its analgesic action is not through opiate receptors. Although the precise mechanism of antinociception is not known it is thought to involve inhibition of synaptosomal uptake of dopamine, norepinephrine and serotonin (VonVoigtlander *et al.,* 1983; Rosland and Hole, 1990; Mather *et al.,* 2001). Previous *in vitro* and *in vivo* studies with nefopam enantiomers have shown that (+)-nefopam has more potent analgesic and dopamine, norepinephrine and serotonin uptake inhibitory properties than (-)-nefopam with the order of potency given as (+)-nefopam > (±)-nefopam > (-)-nefopam (Fasmer *et al.,* 1987; Rosland and Hole, 1990; Mather *et al.,* 2001).

### Summary of the Invention

According to this invention, novel compounds are of general formula (1): 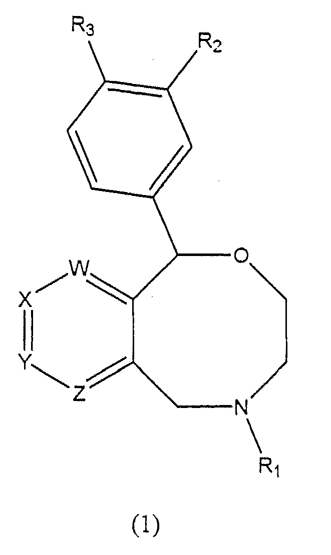 wherein
R₁ is H, C₁-C₆alkyl, optionally substituted with F or C₃-C₆ cycloalkyl or C₂-C₆ alkenyl;
either R₂ and R₃ are the same or different and are H, a halogen, CN, CF₃, C₁-C₆alkyl or OR₁, or R₂ and R₃ form a five or six membered ring which may be carbocyclic, heterocyclic (containing 1-2 heteroatoms taken from O, N or S), aromatic (such as in naphthalene for example), heteroaromatic (containing 1-2 heteroatoms taken from O, such as in benzofuran for example, N as in quinoline, isoquinoline and quinazoline for example); and
W, X, Y or Z are each N, CH or CR₄.

The case where W = X = Y = Z = CH is specifically excluded. When W is N or R₄, X = Y = Z = CH; when X is N or R₄, W = Y = Z = CH; when Y is N or R₄, W = X = Z = CH; and when Z is N or R₄, W = X = Y = CH.

R₄ is halogen, CF₃, CN, OR₇, SO₂N(R₆)₂ (where each R₆ is the same or different), COR₆, CO₂R₆, CON(R₆)₂ (where R₆ maybe the same or different), NR₁COR₅, NR₁SiO₂R₅, NR₁CO₂R₅, NR₁CON(R₆)₂ (where each R₆ is the same or different), OC₁-C₆ alkyl optionally substituted with R₄, C₁-C₆ alkyl optionally substituted with R₄, C₃-C₆ cycloalkyl optionally substituted with R₄, C₂-Cₛ alkenyl optionally substituted with R₄ , C₂-C₆ alkynyl optionally substituted with R₄, and aryl optionally substituted with R₄, R₄ may also be a five or six membered aromatic heterocycle containing 1-4 heteroatoms selected from N (such as in pyrrole, pyridine, diazoles, diazines, triazoles, triazines or tetrazoles for example) and O (such as in furan, oxazoles, isoxazoles or oxadiazoles for example), Such rings can be linked either through carbon or nitrogen.

R₅ is C₁-C₆alkyl, C₂-C₆alkenyl, C₂-C₆ alkynyl, C₃-C₆cycloalkyl, aryl or heteroaryl.

R₆ is H, C₁-C₆alkyl, C₂-C₆alkenyl, C₂-C₆ alkynyl, C₃-C₆cycloalkyl, aryl or heteroaryl.

R₇ is aryl or heteroaryl.

Salts, solvates and polymorphs of these compounds are also included .

Compounds of the invention are useful as therapeutic agents. Further, in compounds of formula (1), those wherein R₄ is a halogen atom such as Br are useful as intermediates.

### Description of Preferred Embodiments

It will be appreciated that the compounds according to the invention contain an asymmetrically substituted carbon atom. The presence of this asymmetric centre in a compound of formula (1) can give rise to stereoisomers, and in each case the invention is to be understood to extend to all such stereoisomers, including enantiomers and diastereomers, and mixtures including racemic and non-racemic mixtures thereof.

As used in this specification, alone or in combination, the term "C₁-C₆ alkyl" refers to straight or branched chain alkyl moiety having from one to six carbon atoms, including for example, methyl, ethyl, propyl, isopropyl, butyl, *tert-*butyl, pentyl, hexyl and the like.

The term "C₂-C₆ alkenyl" refers to a straight or branched chain alkyl moiety having two to six carbon atoms and having in addition one double bond, of either E or Z stereochemistry where applicable. This term would include for example, vinyl, 1-propenyl, 1- and 2- butenyl, 2- methyl-2-propenyl etc.

The term "C₂-C₆ alkynyl" refers to a straight or branched chain alkyl moiety having two to six carbon atoms and having in addition one triple bond. This term would include for example, ethynyl, 1-propargyl, 1- and 2- butynyl etc.

The term "C₃-C₆ cycloalkyl" refers to a saturated alicyclic moiety having from three to six carbon atoms and includes for example cyclopropyl, cyclobutyl, cyclopentyl, cyclohexyl and the like.

The term "aryl" means an optionally substituted phenyl or naphthyl group.

The term "carbocyclic" refers to a saturated alicyclic moiety having five or six carbon atoms and includes for example benzofused cyclopentyl and cyclohexyl and the like.

The term "heterocyclic" refers to a saturated heterocyclic moiety having from five or six atoms but containing one or more heteroatom from the group N, O, S and includes for example benzofused pyrrolidinyl, tetrahydrofuranyl, piperidinyl, dioxalane and the like.

The term "heteroaromatic" refers to aromatic ring systems of five or six atoms or which at least one atom is selected from the group, O, N, or S and includes for example benzofused furanyl, thiophenyl, pyridyl, indolyl, pyridazinyl, piperazinyl, pyrimidinyl and the like.

The term "halogen" means fluorine, chlorine, bromine or iodine.

Compounds of the general formula (1) may be prepared by any suitable method known in the art and/or by the processes described below. It will be appreciated that where a particular stereoisomer of formula (1) is required, the synthetic processes described herein may be used with the appropriate homochiral starting material and/or isomers maybe resolved from mixtures using conventional separation techniques (e.g. HPLC).

The compounds according to the invention may be prepared by the following process. In the description and formulae below the groups R₁, R₂, R₃, R₄, R₅, R₆, W, X, Y and Z are as defined above, except where otherwise indicated. It will be appreciated that functional groups, such as amino, hydroxyl or carboxyl groups, present in the various compounds described below, and which it is desired to retain, may need to be in protected form before any reaction is initiated. In such instances, removal of the protecting group may be the final step in a particular reaction. Suitable protecting groups for such functionality will be apparent to those skilled in the art. For specific details see "Protective Groups in Organic Synthesis", Wiley Interscience, T W Greene, PGM Wuts.

A process required for preparing compounds of general formula (1), where W, X, Y or Z are N or C-Br comprises acid (for instance p-toluenesulphonic acid) cyclisation of the diol of general formula (2) which can in turn be obtained by reduction of the ketone (3) with a suitable reducing agent.

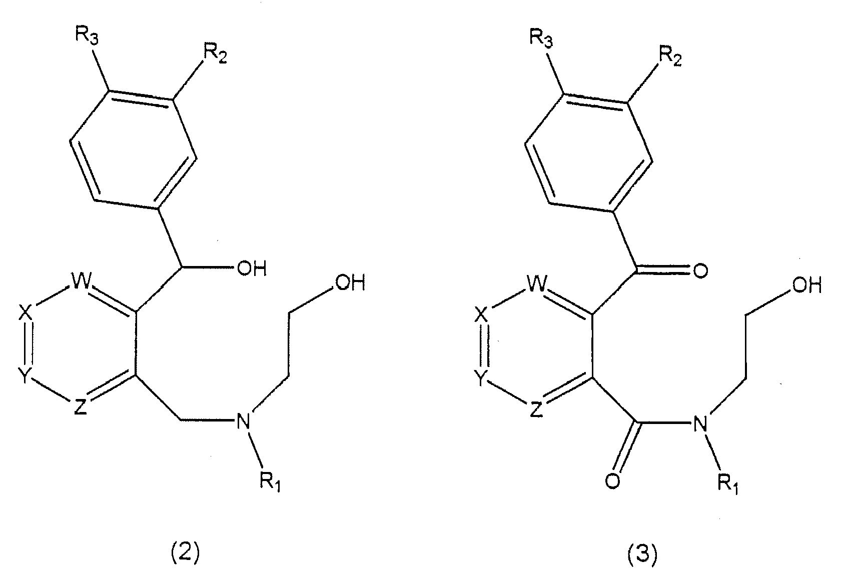

Reduction of a keto amide of general formula (3) can be carried out with reagents well known to those familiar in the art of synthetic organic chemistry. An example of a highly reactive reducing agent is lithium aluminium hydride, although reagents based on borane (e.g. borane.tetrahydrofuran complex) or modified sodium borohydride reduction (e.g. with a nickel or cobalt salt enhancer) are equally effective.

Equally, reduction of the ketone in (3), for example with sodium borohydride, followed by acid cyclisation, for example with p-toluenesulphonic acid, then ultimate reduction of the amide group, for example with borane, also leads to compounds of general formula (1).

Ketones of general formula (3) can be prepared by condensation of a carboxylic acid of general formula (4) or an active derivative thereof, with an amine of formula (5). Active derivatives of acids of formula (4) include for example acid anhydrides or acid halides, such as acid chlorides.

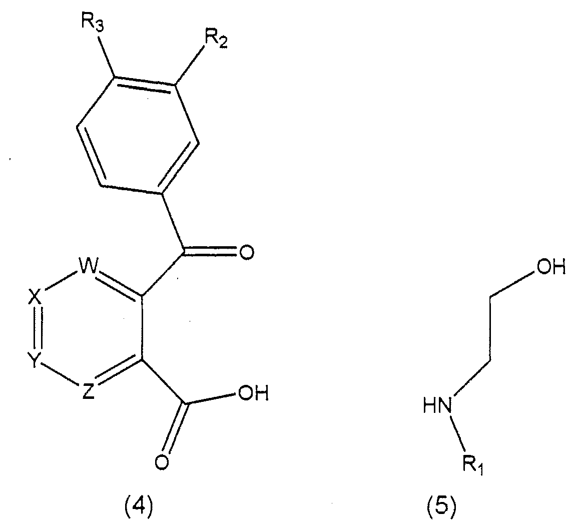

The coupling reaction may be performed using standard conditions for amidation reactions of this type. Thus, the reaction may be achieved in a solvent, for example an inert organic solvent such as an ether, e,g, a cyclic ether such as tetrahydrofuran, an amide e.g. a substituted amide such as dimethylformamide, or a halogenated hydrocarbon such as dichloromethane at a low temperature e.g. -30°C to ambient temperature, such as -20°C to 0°C, optionally in the presence of as base, e.g. an organic base such as an amine, e.g. triethylamine or a cyclic amine such as *N*-methylmorpholine. Where an acid of formula (4) is used directly, the reaction may additionally be performed in the presence of a condensing agent, for example a diimide such as *N*,*N*'-dicyclohexylcarbodiimide, advantageously in the presence of a triazole such as 1-hydroxybenzotriazole, Alternatively, the acid may be reacted with a chloroformate, for example ethyl chloroformate, prior to reaction with the amine of formula (5).

Acids of general formula (4) are prepared by Friedel-Crafts acylation of an arene of general formula (6) with an anhydride of formula (7). This reaction is carried out in an inert solvent (such as dichloromethane) in the presence of a Lewis acid catalyst (such as aluminium trichloride).

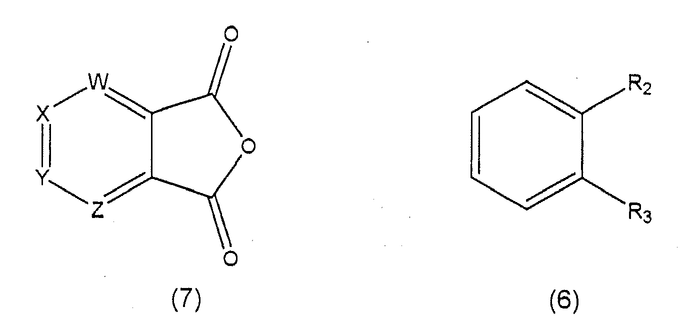

It is well recognised by those skilled in the art that such reactions may provide mixtures of products and in turn that these mixtures can often be separated by tradition flash column chromatography. For example, where Y = C-Br and W = X = Z = CH and R₂ and R₃ are H, Friedel-Crafts acylation under aluminium trichloride catalysis provides two isomeric bromides (4a) and (4b). These can be readily separated by column chromatography and independently progressed to compounds of general formula (1), wherein X or Y are C-Br, by the route described above.

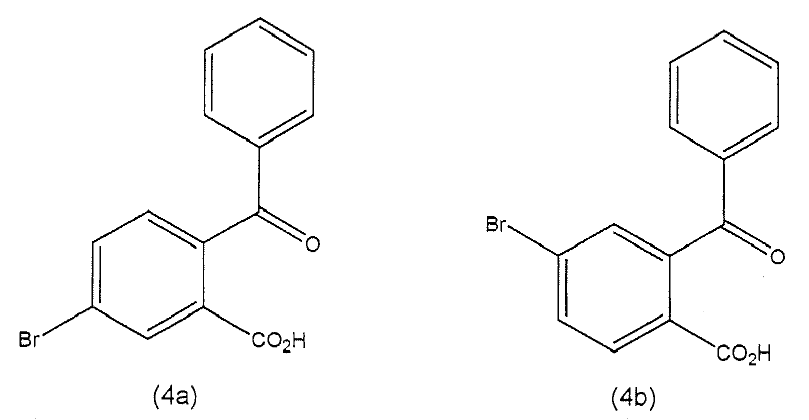

Compounds of general formula (1) where either W, X, Y or Z is CR₄ and R₄ is a halogen such as Br (1a) represent flexible intermediates that may be used for the preparation of other compounds of general formula (1). For instance, compounds of general formula (1) where either W, X, Y or Z is CR₄ and R₄ is Br can be smoothly converted into the corresponding nitrile (1b; R₄ = CN) either by reaction with cuprous cyanide in a dipolar aprotic solvent such as *N-*methylpyrrolidinone (NMP) or under palladium-catalysed conditions (Scheme 1).

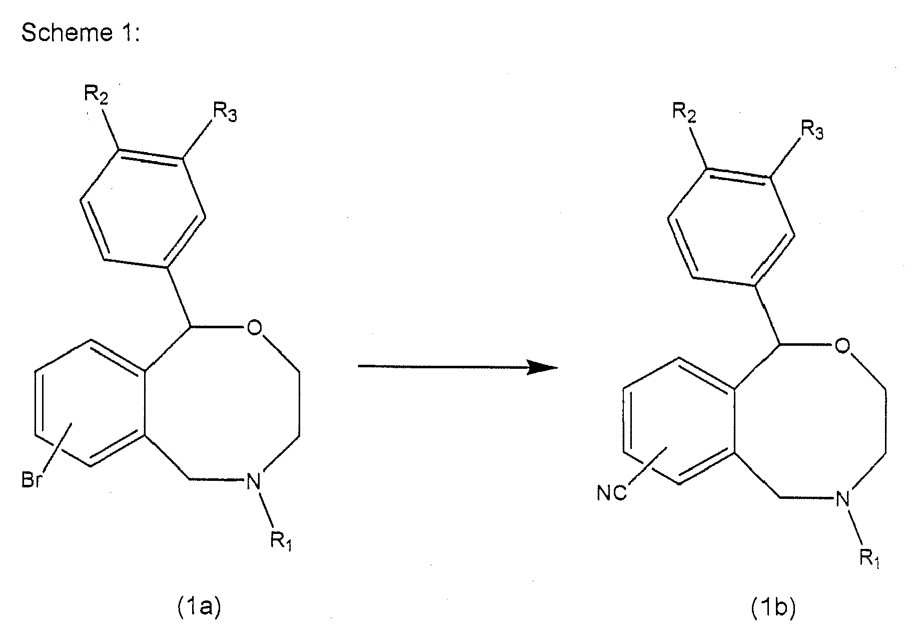

The nitrile of general formula (1) where either W, X, Y or Z is CR₄ and R₄ is CN (1 b) can be readily converted, by hydrolysis, into the primary amide (1c, R₄ = CONH₂), esters and the corresponding carboxylic acid (1 d, CO₂R₁) or into the corresponding tetrazole (1e) by treatment with a suitable azide donor such as sodium azide or trimethylsilylazide (Scheme 2).

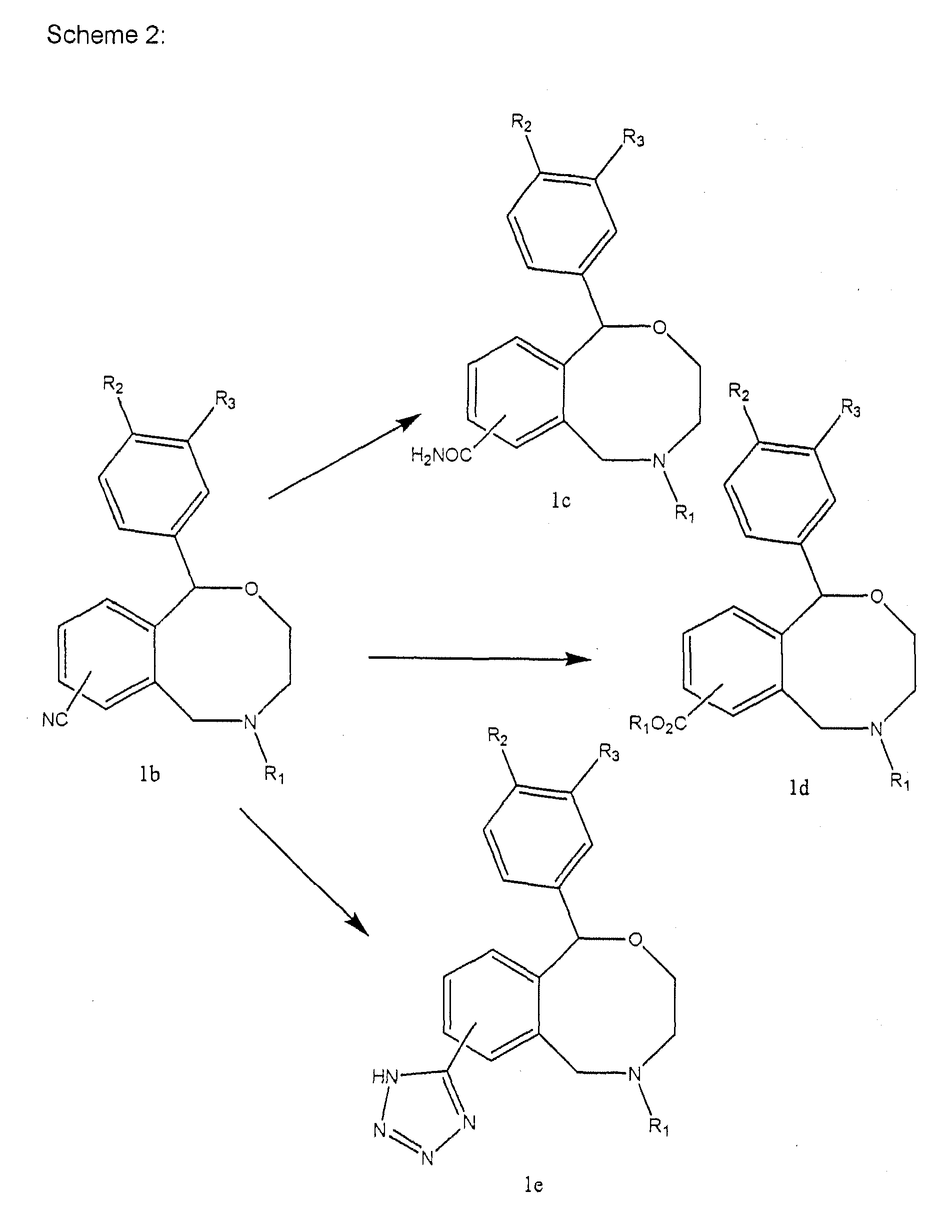

In addition, compounds of general formula (1) where either W, X, Y or Z is CR₄ and R₄ is a halogen such as Br (1a) can be lithiated with n-, sec-, or *tert-*butyllithium in an inert organic solvent such as an ether, e.g. a cyclic ether such as tetrahydrofuran at very low temperature, e.g. -78°C. Treatment with either a carbon (e.g. carbon dioxide, *N*,*N-*dimethyl formamide or paraformaldehyde), sulphur (e.g. SO₂Cl₂, followed by amidation, such as with ammonia) or nitrogen (diphenylphosphoryl azide, followed by reduction, such as with REDAL) provides access, by subsequent derivatisation to derivatives where R₄ is CO₂R₁; CON(R₁)₂ (where each R₁ is the same or different); CH₂OR₁ (1f), SO₂N(R₁)₂ (1g, where each R₁ is the same or different); and NR₁COR₅; NR₁SO₂R₅ (1h); NR₁CO₂R₅; NR₁CON(R₁)₂ (where each R₁ is the same or different). Examples are given in Scheme 3.

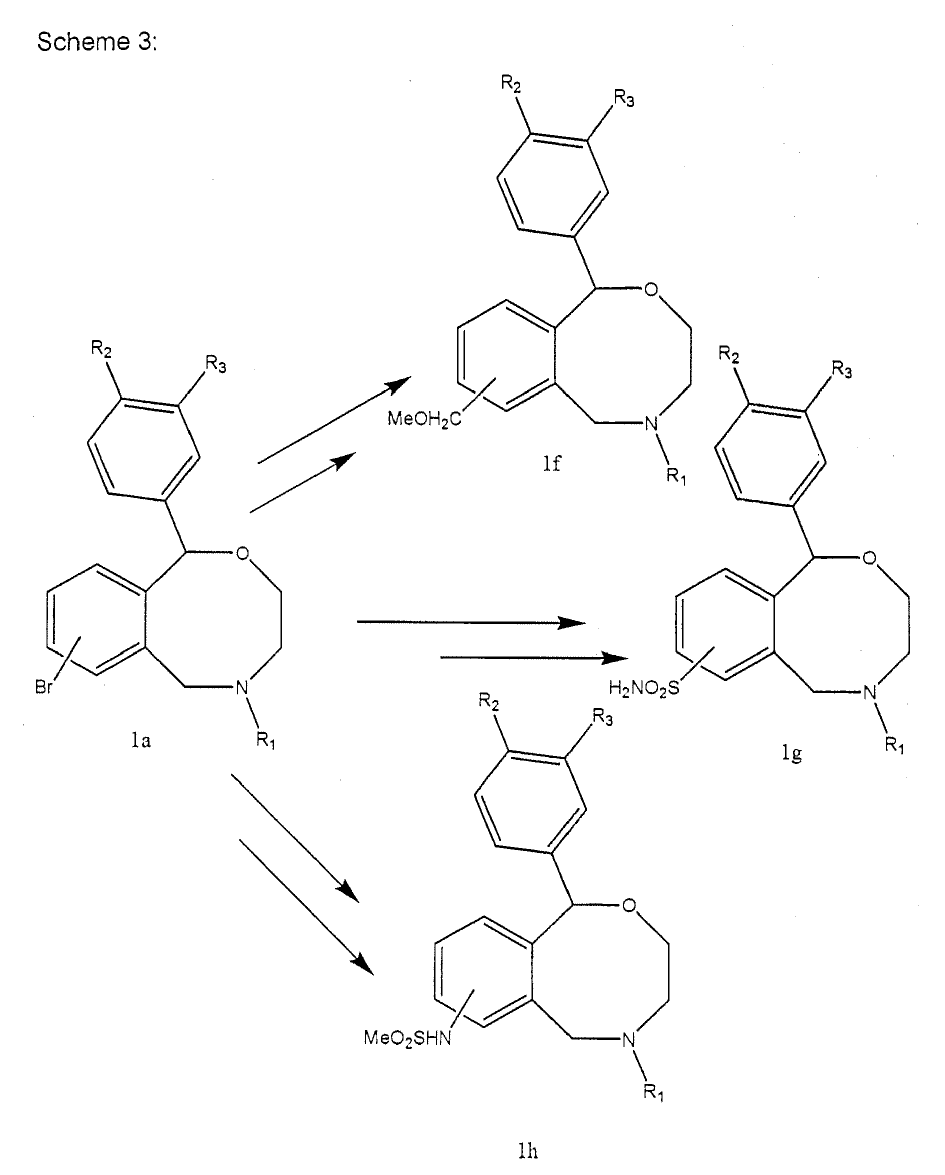

In addition, compounds of general formula (1) where either W, X, Y or Z is CR₄ and R₄ is a halogen such as Br (1 a) can undergo palladium-catalysed coupling reactions with carbon-based coupling partners. Thus, compounds of general formula (1) where either W, X, Y or Z is CR₄ and R₄ is a halogen such as Br can be coupled to alkenes of a general type CH₂=CHR₄ under Heck conditions, alkynes of a general type CH=CHR₄ under Sonogoshira conditions, or metalloheterocycles e.g. where the metal is tin, under Stille coupling conditions. This gives access to compounds where either W, X, Y or Z can be C₂-C₆ alkenyl substituted with R₄ (1 i) C₂-C₆ alkynyl substituted with R₄ (1j) and where R₄ is a five membered aromatic heterocycle containing 1-4 heteroatoms taken from N (such as in pyrrole, diazoles, triazoles or tetrazoles for example) and O (such as in furan (1k), oxazoles or oxadiazoles for example). Such coupling reactions ensure that chains and rings are linked either through carbon. Examples are in Scheme 4.

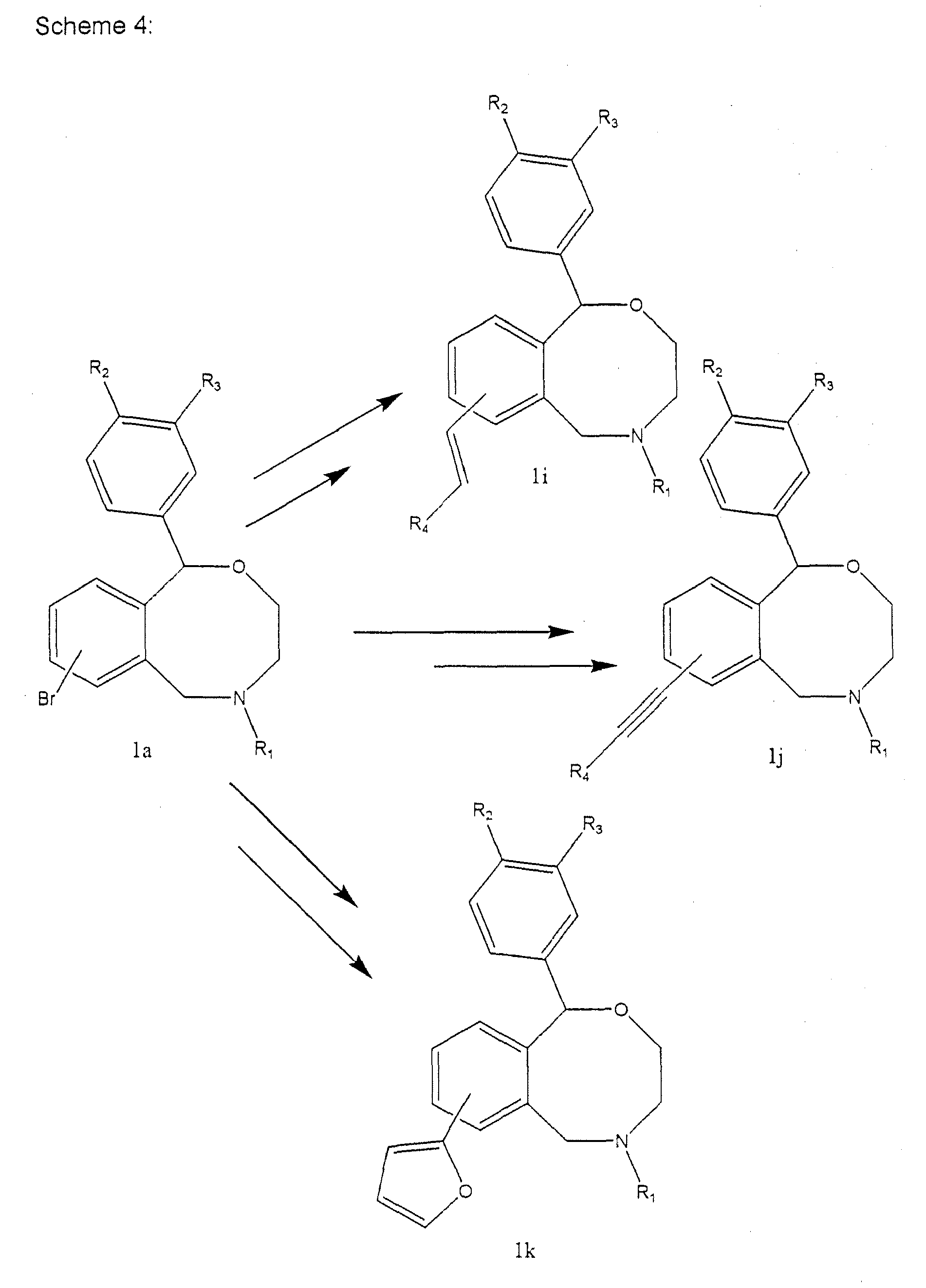

In addition to the examples described above, additional compounds of formula (1) may be prepared by interconversion of other compounds of formula
(1). Thus, for example, a compound of formula (11) wherein R₄ is a C₁₋₆ alkyl group may be prepared by hydrogenation (using palladium on carbon in suitable solvent, such as an alcohol - e.g. ethanol) of a compound of formula (1i) wherein R₄ is a C₂₋₆ alkenyl group (e.g. as in Scheme 5).

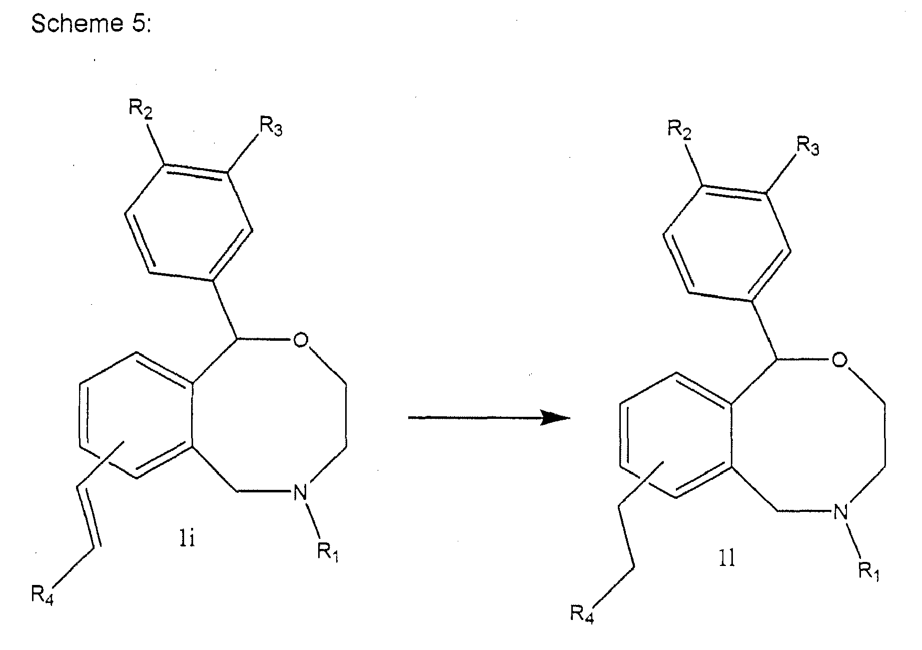

Any mixtures of final products or intermediates obtained can be separated on the basis of the physico-chemical differences of the constituents, in known manner, into the pure final products or intermediates, for example by chromatography, distillation, fractional crystallization, or by formation of a salt if appropriate or possible under the circumstances.

The compounds according to the invention exhibit *in vitro* inhibiting activities with respect to monoamine (i.e. noradrenaline, serotonin and dopamine) reuptake, The activity and selectivity of the compounds may be determined by use of an appropriate monoamine reuptake assay.

This invention also relates to a method of treatment for patients (including man and/or mammalian animals raised in the dairy, meat or fur industries or as pets) suffering from disorders or diseases which can be attributed to monoamine reuptake as previously described, and more specifically, a method of treatment involving the administration of the monoamine reuptake inhibitor of formula (1) as the active constituents.

Accordingly, the compounds of formula (1) can be used among other things in the treatment of pain and emesis but also may find utility in a range of other therapeutic indications such as depression, post traumatic stress disorders, attention deficit disorders, obsessive compulsive disorders, pre-menstrual syndrome, substance abuse and sexual dysfunction;
a method of management (by which is meant treatment of prophylaxis) of disease or conditions mediated by monoamine reuptake in mammals, in particular in humans, which method comprises administering to the mammal an effective, amount of a compound of formula (1) above, or a pharmaceutically acceptable salt thereof;
and a compound of formula (1) for use in human or veterinary medicine, particularly in the management (by which is meant treatment or prophylaxis) of diseases or conditions mediated by monoamine reuptake; and the use of a compound of formula (1) in the preparation of an agent for the management (by which is meant treatment or prophylaxis) of diseases or conditions mediated by monoamine reuptake.

The disease or conditions referred to above include pain, emesis depression, post traumatic stress disorders, attention deficit disorders, obsessive compulsive disorders, pre-menstrual syndrome, substance abuse and sexual dysfunction.

Compunds of formula (1) may be administered orally, topically, buccally, ocularly, rectally, vaginally, parenterally, intra-nasally, sublingually or by inhalation spray, e.g. in dosage unit formulations containing non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, cattle, sheep, dogs, cats etc, the compounds of the invention are effective in the treatment of humans.

The pharmaceutical composition containing the active ingredient may be in a form suitable for oral use, for example, as tablets, troches, lozenges, aqueous or oily suspensions, dispersible powders or granules, emulsions, hard or soft capsules, or syrups or elixirs. The composition may be in immediate or controlled release form.

Compositions intended for oral use may be prepared according to any method known to the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastointestinal tract and thereby provide a sustained action over a longer period. For example, a time delay material such as glyceryl monostearate or glyeryl distearate may be employed. They may also be coated by the techniques described in the US Patents 4,256,108;4,166,452; and 4,265,874 to form osmotic therapeutic tablets for control release.

Formulations for oral use may also be presented as hard gelatin capsules where in the active ingredient is mixed with an inert solid diluent, for example calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate polyvinylpyrrolidone, gum tragacanth and gum acacia; dispersing or wetting agents may be a naturally occuring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters dervied from fatty acids and a hexitol such a polyoxyethylene with partial esters derived from fatty acids and hexitol anhydrides, for example polyoxyethylene sorbitan monooleate. The aqueous suspensions may also contain one or more preservatives, for example ethyl or n-propyl p-hydroxybenzoate, one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified, for example sweetening, flavouring and colouring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occuring gums, for example gum acacia or gum tragacanth, naturally-occuring phosphatides, for example soya bean, lecithin, and esters or partial esters derived from fatty acids and hexitol anhydrides, for example sorbitan monooleate and condensation products of the said partial esters with ethylene oxide, for example polyoxyethylene sorbitan monooleate. The emulsions may also contain sweetening and flavouring agents.

Syrups and elixirs may be formulated with sweetening agents, for example gycerol, propylene glycol, sorbitol or sucrose. Such formulations may also contain a demulcent, a preservative and flavouring and colouring agents. The pharmaceutical compositions may be in the form of a sterile injectable aqueous or oleagenous suspension, This suspension may be formulated according to the known art using those suitable dispersing or wetting agents and suspending agents which have been mentioned above. The sterile injectable preparation may also be in a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution, In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of formula (1) may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc containing the compounds of Formula (1) are employed. For the purposes of this application, topical application includes mouth washes and gargles.

Dosage levels of the order of from about 0.05 mg to about 140 mg per kilogram of body weight per day are useful in the treatment of the above-indicated conditions (about 2.5 mg to about 7 gms per patient per day). For example, emesis may be effectively treated by the administration of from about 0.01 to 50 mg of the compound per kilogram of body weight per day (about 0.5 mg to about 3.5 gms per patient per day),

The amount of active ingredient that may be combined with the carrier materials to produce a single dosage form will vary depending upon the host treated and the particular mode of administration. For example, a formulation intended for the oral administration of humans may vary from about 5 to about 95 percent of the total composition. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet time of administration, route of administration, rate of excretion, drug combination and the severity of the particular disease undergoing therapy.

The following Examples illustrate the invention.

### Experimental

### 2-Benzoyl-4-bromobenzoic acid (2a) and 2-Benzoyl-5-bromobenzoic acid (2b)

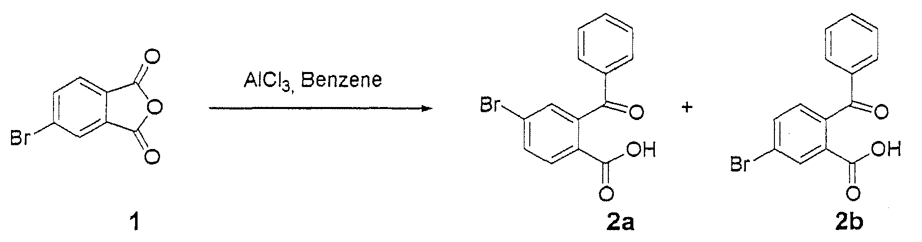

A mixture of 4-bromophthalic anhydride (1) (8,6g, 37,9mmol) and aluminium chloride (10g, 75mmol, 2 equiv.) were heated under reflux for six hours under an atmosphere of nitrogen. The hot reaction mixture was poured into a solution of water: conc. hydrochloric acid (9:1, 200mL) and the aqueous layer extracted with dichloromethane (2 x 150mL). The organic extract was dried over magnesium sulphate, filtered and evaporated under reduced pressure to furnish the crude product as an off white solid. The solid was dissolved in ethanol (80mL) and water added until the solution remained turbid. The mixture was allowed to stand at room temperature for four hours; the precipitate formed was filtered, washed with hexane (2 x 10mL) and dried under suction to furnish compound 2a. Yield 4.1 g, 35%.

¹H nmr (250MHz, CDCl₃); 7.94 (1H, d, *J* 8.0, CHₐᵣ), 7.71 (3H, m, CHₐᵣ), 7.58 (1H, t, *J* 7.5, CHₐᵣ), 7.51 (1H, d, *J* 1.5 CHₐᵣ), 7.44 (2H, t, *J* 7.5, CHₐᵣ), 7.0-6.4 (1H, bs, OH).

The mother liquors were concentrated under reduced pressure to half the volume and stirred at room temperature. The precipitate formed was filtered, washed with hexane (2 x 10mL) and dried under suction to provide 2b. Yield 4.8g, 42%
¹H nmr (250MHz, CDCl₃); 8.70-8.30 (1H, bs, OH), 8.20 (1H, d, *J* 1.5, CHₐᵣ), 7.80 (2H, dd, *J* 8.0, 1.5, CHₐᵣ), 7.71 (2H, m, CHₐᵣ), 7.58 (1H, m, CHₐᵣ), 7.44 (2H, m, CHₐᵣ), 7.26 (1H, d, *J* 8.0 CHₐᵣ).

### N-(2-hydroxyethyl)-N-methyl-2-benzoyl-4-bromobenzamide (3a)

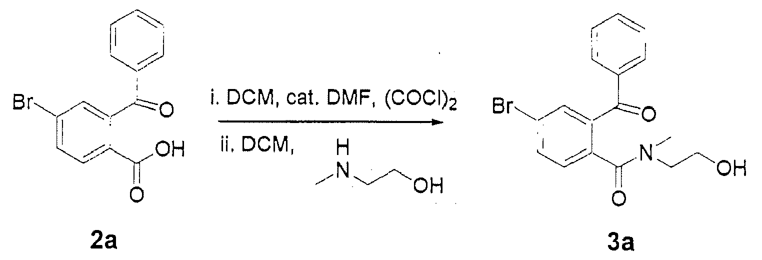

A solution of 2M oxalyl chloride (3.69mL, 7.38mmol, 1.1 equiv.) in dichloromethane was added dropwise to a suspension of compound **2a** in dichloromethane (12mL) and catalytic *N*,*N*-dimethylformamide (2 drops) at room temperature under a nitrogen atmosphere. Gas evolution was rapid and as the reaction proceeded the solid dissolved in the dichloromethane. After 2.5 hours the solvent was removed under reduced pressure and the resulting solid co-evaporated with dichloromethane (2 x 20mL) to remove traces of excess oxalyl chloride. The crude acid chloride was dissolved in dichloromethane (15mL) and added dropwise to a solution of N-methylaminoethanol (593µL, 7,38mmol, equiv.) and triethylamine, (1.03mL, 7.38mmol, 1.1 equiv) in dichloromethane (15mL) cooled to 0°C in an ice bath. The resulting solution was stirred at room temperature for 3 hours, quenched with saturated aqueous sodium dicarbonate (20mL) and separated. The aqueous layer was extracted with dichloromethane (30mL) and the combined organic fractions washed with brine (20mL), dried over magnesium sulfate and filtered. The solvent was removed under reduced pressure and the crude product purified by column chromatography, eluting with ethyl acetate : hexane (4:1), followed by ethyl acetate (100%) as product eluted. Compound 3a was furnished as a semi solid. Yield 2.23g, 92%. The product exists are a mixture of rotomers in a 3:2 ratio.
¹H nmr (250MHz, CDCl₃); 7.85 (2H, m, CHₐᵣ), 7.75-7.62 (3H, m, CHₐᵣ), 7.34 (0.6H, *J* 8.0, CHₐᵣ), 7.33 (0.4H, *J* 8.0, CHₐᵣ), 3.83 (1.2H, t, *J* 4.5, CH₂OH), 3.76 (0.8H, t, *J* 4.5, CH₂OH), 3.59 (2H, t, *J* 4.5, NCH₂), 3.08 (0.4H, s, NCH₃)2.99 (0.6H, s, NCH₃), 2.45 (1H, bs, OH). ***N*-(2-hydroxyethyl)-*N*-methyl-2-benzoyl-5-bromobenzamide (3b)**

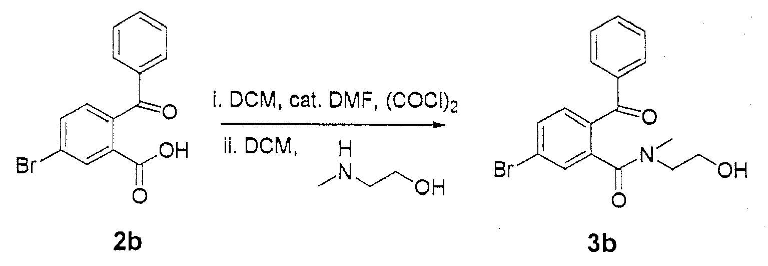

A solution of oxalyl chloride (6.5mL, 74.3mmol, 1.1 equiv.) in dichloromethane (150mL) was added dropwise to a suspension of compound **2a** (20.6g, 67.5mmol, ratio of 3:1 of **2a:2b** respectively) in dichloromethane (50mL) and catalytic *N*,*N*-dimethylformamide (4 drops) at room temperature under a nitrogen atmosphere. Gas evolution was rapid and as the reaction proceeded the solid dissolved in the dichloromethane. After 2.5 hours the solvent was removed under reduced pressure and the resulting solid co-evaporated with dichloromethane (2 x 50mL) to remove traces of excess oxalyl chloride. The crude acid chloride was dissolved in dichloromethane (100mL) and added dropwise to a solution of N-methylaminoethanol (6mL, 74.3mmol, 1.1 equiv.) and triethylamine, (10.4mL, 74.3mmol, 1.1 equiv) in dichloromethane (150mL) cooled to 0°C in an ice bath. The resulting solution was stirred at room temperature for 3 hours, quenched with saturated brine (100mL) and separated. The aqueous layer was extracted with dichloromethane (2 x 50mL) and the combined organic fractions washed with brine (100mL), dried over magnesium sulfate and filtered. The solvent was removed under reduced pressure and the crude product purified by column chromatography, eluting with ethyl acetate : hexane (4:1) to elute compound **3b.** Yield 6.2g, 25%. Eluting with ethyl acetate (100%) furnished compound **3a** as a semi solid. Yield 14.6g, 60%. The product exists are a mixture of rotomers in a 3:2 ratio.
¹H nmr (250MHz, CDCl₃); 7.78 (1.8H, m, CHₐᵣ), 7.60 (2.7H, m, CHₐᵣ), 7.49 (2.5H, CHₐᵣ), 7.45 (0.4H, d, *J* 8.5, CHₐᵣ), 7.37 (0.6H, d, *J* 8.5, CHₐᵣ), 3.86 (1.2H, m, CH₂OH), 3.76 (0.8H, m, CH₂OH), 3.61 (2H, m, NCH₂), 3.08 (0.4H, s, NCH₃), 3.00 (0.6H, s, NCH₃).

### N-(2-hydroxyethyl)-N-methyl-4-bromo-2-(1-hydroxy-1-phenyl)methyl benzylamine (4a)

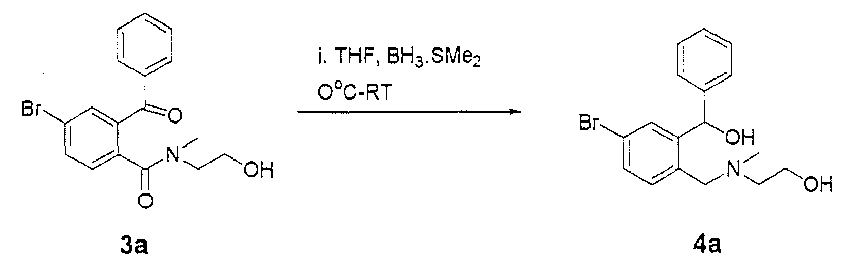

Amide 3a (13.8g, 38mmol) was dissolved in tetrahydrofuran (75mL) and cooled to 0°C in an ice bath. A 2M solution of borane dimethylsulfide complex (84mL, 168mmol, 4.4 equiv.) was added dropwise and the resulting solution stirred at room temperature for 17 hours. The reaction was carefully quenched with 6M hydrochloric acid solution (84mL) and the resulting solution heated under reflux for 1 hour. Tetrahydrofuran was removed under reduced pressure and remaining solution diluted with water (70mL) and extracted with diethyl ether (2 x 100mL). The aqueous layer was basified with 3.75M sodium hydroxide solution and extracted with ethyl acetate (2 x 200mL). The combined ethyl acetate extracts were dried over magnesium sulfate, filtered and evaporated under reduced pressure to furnish the desired product 4a as a colourless glass. Yield 10.2g, 77%.
¹H nmr (250MHz, CDCl₃); 7.38-7.27 (7H, m, CHₐᵣ), 7.08 (1H, d, *J* 8.0, CHₐᵣ), 5.83 (1H, s, CHOH), 3.70-3.65 (2H, m, OCH₂), 3.41 (1H, d, *J* 12.5, ArCHₐH_{b}N), 3.29 (1H, d, *J* 12.5, ArCHₐH_{b}N), 2.58-2.55 (2H, m, NCH₂), 2,21 (3H, s, NCH₃).

### N-(2-hydroxyethyl)-N-methyl- 5-bromo-2-(1-hydroxy-1-phenyl)methyl benzylamine (4b)

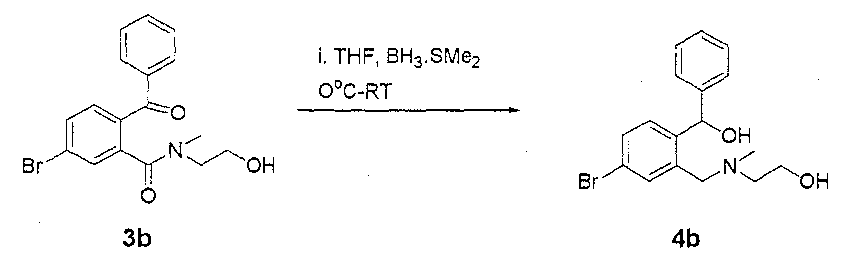

Amide **3b** (7.4g, 20.4mmol) was dissolved in tetrahydrofuran (40mL) and cooled to 0°C in an ice bath. A 2M solution of borane dimethylsulfide complex (45mL, 90mmol, 4.4 equiv.) was added dropwise and the resulting solution stirred at room temperature for 17 hours. The reaction was carefully quenched with 6M hydrochloric acid solution (45mL) and the resulting solution heated under reflux for 1 hour. Tetrahydrofuran was removed under reduced pressure and the remaining solution was diluted with water (45mL) and extracted with diethyl ether (3 x 50mL), The aqueous layer was basified with 3.75M sodium hydroxide solution and extracted with ethyl acetate (2 x 100mL). The combined ethyl acetate extracts were dried over magnesium sulfate, filtered and evaporated under reduced pressure to furnish the desired product 4b as a colourless glass. Yield 6,5g, 91 %.
¹H nmr (250MHz, CDCl₃); 7.41-7.27 (7H, m, CHₐᵣ), 7.06 (1H, d, *J* 8.0, CHₐᵣ), 5.86 (1H, s, CHOH), 3.72-3.66 (2H, m, OCH₂), 3.44 (1H, d, *J* 12.5, ArCHₐH_{b}N), 3.32 (1H, d, *J* 12.5, ArCHₐH_{b}N), 2.59 (2H, m, NCH₂), 2.23 (3H, s, NCH₃).

### 9-Bromo-5-methyl-1-phenyl-1,3,4,6-tetrahydro-5H-benz[f]-2,5-oxazocine (5a)

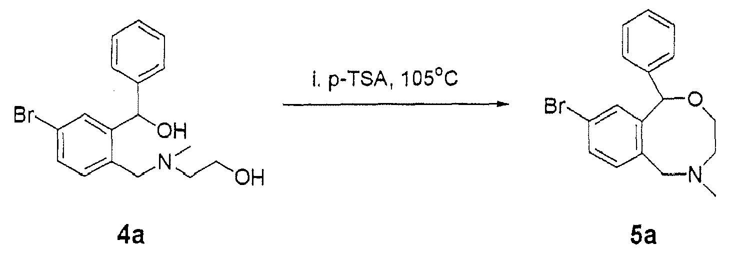

Diol **4a** (14.6g, 41.6mmol) was dissolved in toluene (115mL) and *para*toluenesulfonic acid monohydrate (11.9g, 62.4mmol, 1.5equiv.) added. The toluene was removed under reduced pressure and the resulting oil heated at 105°C for 4 hours. On cooling the oil was suspended in water (100mL) and basified with 3.75M sodium hydroxide solution. The aqueous layer was extracted with ethyl acetate (2 x 200mL), dried over magnesium sulfate, filtered and evaporated under reduced pressure to furnish the product **5a** as pale brown oil. Yield 9.25g, 67%.
¹H nmr (250MHz, CDCl₃); 7.38-7.25 (5H, m, CHₐᵣ), 7.11 (2H, t, *J*8.0, CHₐᵣ), 5.72 (1H, s, CHO), 4.82 (1H, d, *J* 13.0, ArCHₐH_{b}), 4.19 (1H, dt, *J* 3.0, 8.0, OCHₐH_{b}), 3.82 (1H, ddd, *J* 3.0, 6.0, 13.0, OCHₐH_{b}), 3.62 (1H, d, *J* 13.0, ArCHₐH_{b}), 2.81 (1H, m NCHₐH_{b}), 2.61 (1H, ddd, *J* 3.0, 8.0, 13.0, NCHₐH_{b}), 2.43 (3H, s, CH₃).

### 8-Bromo-5-methyl-1-phenyl-1,3,4,6-tetrahydro-5H-benz[f]-2,5-oxazocine (5b)

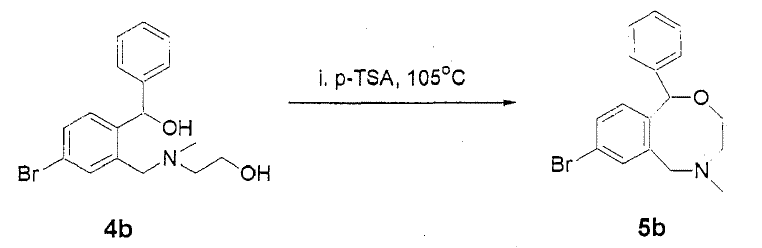

Diol **4b** (6.5g, 18,6mmol) was dissolved in toluene (50mL) and paratoluenesulfonic acid monohydrate (5.3g, 27.8mmol, 1.5equiv.) added. The toluene was removed under reduced pressure and the resulting oil heated at 105°C for 4 hours. On cooling the oil was dissolved in 3.75M sodium hydroxide solution and extracted with ethyl acetate (2 x 80mL), dried over magnesium sulfate, filtered and evaporated under reduced pressure to furnish the crude product. The crude product was purified by dry flash chromatography eluting with ethyl acetate (100%, followed by ethyl acetate:methanol (5%)). Fractions containing product were combined and evaporated under reduced pressure to furnish **5b** as pale brown oil. Yield 3.6g, 58%.
¹H nmr (250MHz, CDCl₃); 7.37-7.22 (7H, m, CHₐᵣ), 6.86 (1H, d, *J* 8.0, CHₐᵣ), 5.74 (1H, s, CHO), 4.79 (1H, d, *J* 13.0, ArCHₐH_{b}), 4.16 (1H, dt, *J* 3.0, 8.0, OCHₐH_{b}), 3.85 (1H, ddd, *J* 2.0, 6.0, 13.0, OCHₐH_{b}), 3.61 (1H, d, *J* 13.0, ArCHₐH_{b}), 2.81 (1H, ddd, *J* 2.0*,* 8.0, 13.0, NCHₐH_{b}), 2.63 (1H, ddd, *J* 3.0, 6.0, 13.0, NCHₐH_{b}), 2.46 (3H, s, CH₃).

### 9-Cyano-5-methyl-1-phenyl-1,3,4,6-tetrahydro-5H-benz[f]-2,5-oxazocine (6a)

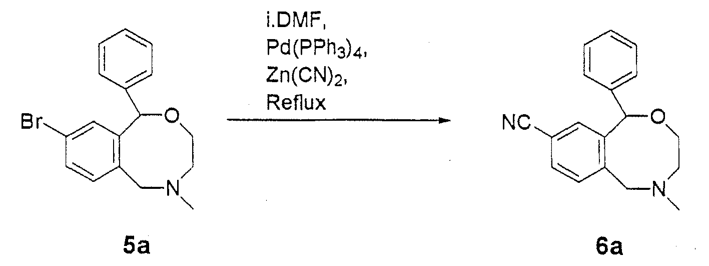

Bromo analogue **5a** (0.2g, 0.6mmol), Zn(CN)₂ (53mg, 0.6mmol), and Pd(PPh₃)₄ (34mg, 0.03mmol), were dissolved in degassed anhydrous DMF (3mL) under a N₂ atmosphere. The mixture was refluxed under N₂ for 24 hours. The mixture was allowed to cool to room temperature, filtered through celite and washed through with DCM (50ml). The filtrate was then quenched with water (10ml) and solvent extracted. The organic extract was dried over MgSO₄, filtered and solvent removed under reduced pressure. The crude product was purified by dry flash chromatography eluting with DCM: MeOH (98:2). Fractions containing the product were combined and evaporated under reduced pressure to produce **6a** as pale brown oil, Yield 108mg, 63%.
¹H nmr (250MHz, CDCl₃); 7.51 (1H, dd, *J* 2.0, 8.0, CHₐᵣ), 7.35-7.23 (7H, m, CHₐᵣ), 5.79 (1H, s, CHO), 4.90 (1H, d, *J* 13.0, ArCHₐH_{b}), 4.25-4.16 (1H, m, OCHₐH_{b}), 3.86 (1H, ddd, *J* 3.0, 6.0, 13,0, OCHₐH_{b}), 3.71 (1H, d, *J* 13.0, ArCHₐH_{b}), 2.78(1H, ddd, *J* 3.0, 8.0, 13.0, NCHₐH_{b}), 2.68 (1H, ddd, *J* 3.0*,* 5,0, 13.0, NCHₐH_{b}), 2.45 (3H, s, CH₃).

### 8-Cyano-5-methyl-1-phenyl-1,3,4,6-tetrahydro-5H-benz[f]-2,5-oxazocine (6b)

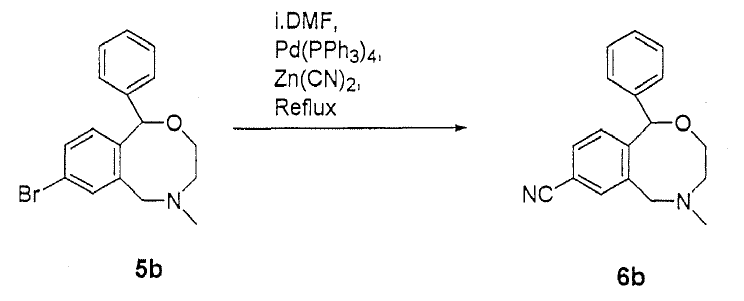

Bromo analogue **5b** (0.2g, 0.6mmol), Zn(CN)₂ (53mg, 0.6mmol), and Pd(PPh₃)₄ (34mg, 0.03mmol), were dissolved in degassed anhydrous DMF (3mL) under a N₂ atmosphere. The mixture was refluxed under N₂ for 24 hours. There is no change in Rf value for compound **6b** from the starting material **5b**. Completion of the reaction can only be determined by ¹H nmr. The mixture was allowed to cool to room temperature, filtered through celite and washed through with DCM (50ml). The filtrate was quenched with water (10ml) and solvent extracted. The organic extract was dried over MgSO₄, filtered and solvent removed under reduced pressure. The crude product was purified by dry flash chromatography eluting with DCM: MeOH (98:2). Fractions containing the product were combined and evaporated under reduced pressure to produce 6b as pale brown oil. Yield 110mg, 66%.
¹H nmr (250MHz, CDCl₃); 7.46 (2H, td, *J* 2.0, 8.0, CHₐᵣ), 7.31 (5H, m, CHₐᵣ), 7.10 (1H, d, *J* 8.0, CHₐᵣ), 5.76 (1H, s, CHO), 4.97 (1H, d, *J* 13.0, ArCHₐH_{b}), 4.25 (1H, ddd, *J* 3.0, 8.0, 13.0, OCHₐH_{b}), 3.82 (1H, ddd, *J* 3.0, 5.0, 13.0, ArCHₐH_{b}), 3.62 (1H, d, *J* 13.0, NCHₐH_{b}), 2.74(1H, ddd, *J* 2.0, 8.0, 13.0, NCHₐH_{b}), 2.44 (3H, s, CH₃).

### 5-methyl-1-phenyl-1,3,4,6-tetrahydro-5H-benz[f]-2,5-oxazocine-9-carboxamide (7a)

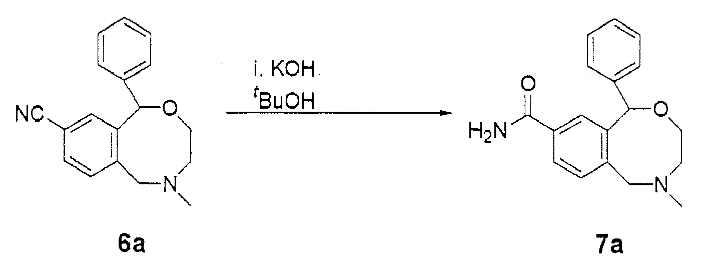

Finely ground potassium hydroxide (130mg, 2.32mmol) in tertiary butanol (4ml) was added to nitrile **6a** (169mg, 0.61 mmol). The solution was heated under reflux for 1.5 hours with stirring. On cooling, the reaction was diluted with brine (12.5ml) and extracted with chloroform (3 x 10ml). The combined organic layers were washed with brine (2 x 5ml), dried over potassium carbonate and concentrated under reduced pressure to yield a yellow solid. Flash column chromatography (15%-25% methanol in ethyl acetate) furnished amide **7a** as a cream coloured solid. Yield 195mg, quantitative yield.
IR (νₘₐₓ/cm⁻¹) 1656;
¹H nmr (250MHz, CD₃OD); 7.75 (1H, dd, *J* 7.9, 1.8, CHₐᵣ), 7.60 (1H, d, *J* 1.5, CHₐᵣ), 7.36 (1H, d, *J* 8.0, CHₐᵣ), 7.28 (5H, brs, CHₐᵣ), 5.86 (1H, s, CHO), 5.02 (1H, d, *J* 12.5, ArCHₐCH_{b}), 4.22 (1H, m, OCHₐH_{b}), 3.88 (1H, dq, *J* 12.7, 4.7, 2.5, OCHₐH_{b}), 3.72 (1H, d, *J* 12.5, ArCHₐCH_{b}), 2.76 (1H, m, NCHₐH_{b}), 2.56 (1H, dq, *J* 14.2, 3.1, NCHₐH_{b}), 2.43 (3H, s, NCH₃).

### 5-methyl-1-phenyl-1,3,4,6-tetrahydro-5H-benz[f]-2,5-oxazocine-8-carboxamide (7b)

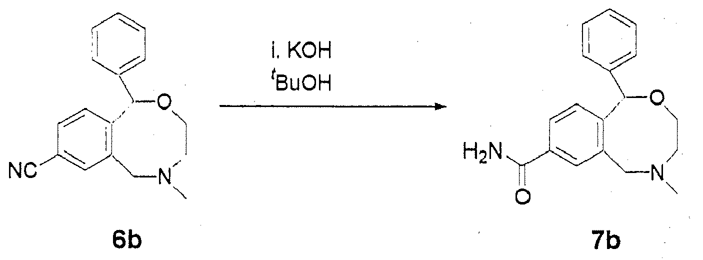

Finely ground potassium hydroxide (87mg, 1.55mmol) in tertiary butanol (4ml) was added to nitrile **6b** (135mg, 0.49mmol). The solution was heated under reflux for 1.5 hours with stirring. On cooling, the reaction was diluted with brine (12.5ml) and extracted with chloroform (3 x 10ml). The combined organic layers were washed with brine (2 x 5ml), dried over potassium carbonate and concentrated under reduced pressure to yield a yellow solid. Flash column chromatography (15%-25% methanol in ethyl acetate) furnished amide **7b** as a cream coloured solid. Yield 140mg, 97%.
¹H nmr (250MHz, CD₃OD); 7.74-7.63 (2H, m, CHₐᵣ), 7.25-7.09 (6H, m, CHₐᵣ), 5.80 (1H, s, CHO), 4.96 (1H, d, *J* 10.1, ArCHₐCH_{b}), 4.25 (1H, d, *J* 10.0, ArCHₐCH_{b}), 3.89-3.65 (2H, m, OCHₐH_{b}), 2.83 (1H, m, NCHₐH_{b}), 2.66 (1H, m, NCHₐH_{b}), 2.53 (3H, s, NCH₃).

### N-(1,1,1-trimethylmethoxycarbonyl)-5-methyl-1-phenyl-1,3,4,6-tetrahydro-5H-benz[f]-2,5-oxazocine-9-methylamine (8a)

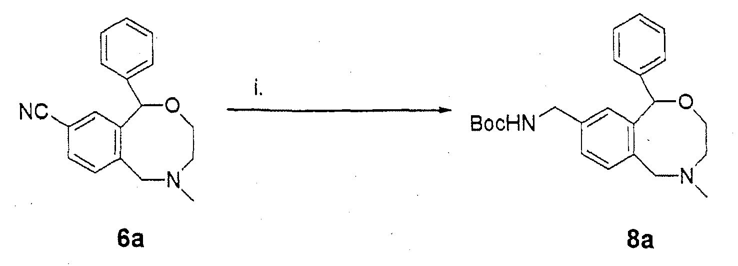

Sodium borohydride (250mg, 6.61 mmol) was cautiously added to a solution of nickel chloride (122mg, 0.94mmol), Boc₂O (412mg, 1.89mmol) and nitrile **6a** (260mg, 0.94mmol) in anhydrous methanol (10ml) at 0°C. Once the vigorous initial reaction had subsided, the mixture was left to stir overnight under a nitrogen atmosphere at room temperature. The methanol was removed under reduced pressure and the resulting precipitate dissolved in ethyl acetate. Saturated aqueous sodium bicarbonate was added, the mixture sonicated and the resulting precipitate filtered. The organic layer was separated from the aqueous which was extracted with ethyl acetate (2 x 10mL). The combined organic layers were dried over sodium sulfate and concentrated under reduced pressure to yield a brown oil. Flash column chromatography (5%-10% methanol in dichloromethane) furnished protected amine **8a** as an orange/brown oil. Yield 141 mg, 39%.
¹H nmr (250MHz, CDCl₃); 7.29-7.15 (7H, m, CHₐᵣ), 6.88 (H, s, CHₐᵣ), 5.74 (1H, s, CHO), 4.79 (1H, d, *J* 13, ArCHₐCH_{b}), 4.19-4.17 (3H, m, CH₂, OCHₐH_{b}), 3.83 (1H, d, *J* 2, 6, 13, ArCHₐCH_{b}), 3.65 (1H, d, *J* 13.0, NCHₐH_{b}) 2.78 (1H, ddd, *J* 2, 8, 15, OCHₐH_{b}), 2.61 (1H, ddd, *J* 2, 6, 15, NCHₐH_{b}), 2.43 (3H, s, CH₃), 1.45 (9H, s, Boc, ^{t}Bu).

### N-(1,1,1-trimethylmethoxycarbonyl)-5-methyl-1-phenyl-1,3,4,6-tetrahydro-5H-benz[f]-2,5-oxazocine-8-methylamine (8b)

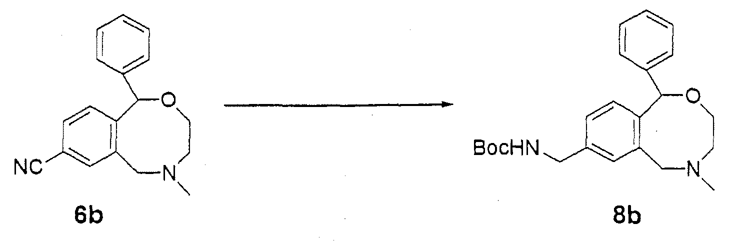

Sodium borohydride (470mg, 12.6mmol) was cautiously added to a solution of nickel chloride (460mg, 3.6mmol), Boc₂0 (780mg, 3.6mmol) and nitrile **6a** (500mg, 1.8mmol) in anhydrous methanol (20ml) at 0°C. Once the vigorous initial reaction had subsided, the mixture was left to stir overnight under a nitrogen atmosphere at room temperature. The methanol was removed under reduced pressure and the resulting precipitate dissolved in ethyl acetate. Saturated aqueous sodium bicarbonate was added, the mixture sonicated and the resulting precipitate filtered. The organic layer was separated from the aqueous which was extracted with ethyl acetate (2 x 10mL). The combined organic layers were dried over sodium sulfate and concentrated under reduced pressure to yield a brown oil. Flash column chromatography (5%-10% methanol in dichloromethane) furnished protected amine **8b** as an orange/brown oil. Yield 260mg, 48%.
¹H nmr (250MHz, CDCl₃); 7.30-7.16 (7H, m, CHₐᵣ), 6.90 (H, s, CHₐᵣ), 5.74 (1H, s, CHO), 4.83 (1H, d, *J* 12.8, ArCHₐCH_{b}), 4.77 (H, brs, NH), 4.23 (3H, m, CH₂, OCHₐH_{b}), 3.87(1H, ddd, *J* 2, 5, 13, ArCHₐCH_{b}), 3.69 (1H, d, *J* 13.0, NCHₐH_{b}) 2.81 (1H, m, OCHₐH_{b}), 2.64(1H, ddd, *J* 3.5, 14NCHₐH_{b}), 2.47(3H, s, CH₃), 1.42 (9H, s, Boc, ^{t}Bu).

### 5-methyl-1,9-diphenyl-1,3,4,6-tetrahydro-5H-benz[f]-2,5-oxazocine (9a)

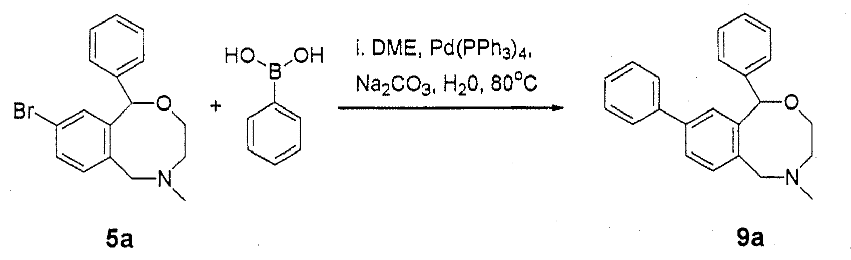

To a solution of **5a** (280mg, 0.84mmol) in dimethoxyethane (6mL) was added tetrakis(triphenylphosphine) palladium (105mg, 10mol%) and the solution stirred at room temperature for 10 minutes under and atmosphere of nitrogen. A solution of sodium carbonate (536mg, 5.0mmol, 6 equiv.) in water (2mL) was added and the biphasic system stirred for 10 minutes. Phenylboronic acid (103mg, 1.1 mmol, 1.3 equiv.) was added and the mixture heated at 80°C for 1.5 hours. The reaction was diluted with dichloromethane (20mL), washed with water (20mL) and the organic layer dried over MgSO₄. Filtration and evaporation under reduced pressure furnished a brown oil. Diethyl ether (25mL) was added and a precipitate formed. After filtration the filtrate was evaporated under reduced pressure and the resulting oil purified by column chromatography, eluting with diethyl ether : methanol (4:1). Fractions containing product were combined, evaporated under reduced pressure to furnish the desired product as a pale yellow oil. Yield 172mg, 61 %.
¹H nmr (250MHz, CDCl₃); 7.52-7.47 (3H, m, CHₐᵣ), 7.43-7.25 (10H, m, CHₐᵣ), 5.87 (1H, s, CHO), 4.88 (1H, d, *J* 12.5, ArCHₐCH_{b}), 4.24 (1H, m, OCHₐCH_{b}), 3.92 (1H, m, OCHₐCH_{b} ), 3.72 (1H, d, *J* 12.5, ArCHₐCH_{b}), 2.89 (1H, m, NCHₐCH_{b}), 2.68 (1H, m, NCHₐCH_{b}), 2.51 (3H, s, NCH₃).

### 5-methyl-1,8-diphenyl-1,3,4,6-tetrahydro-5H-benz[f]-2,5-oxazocine (9b)

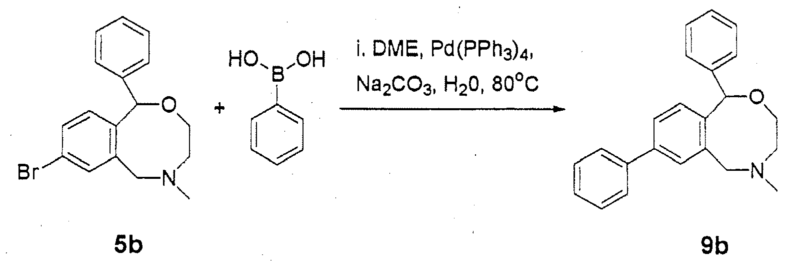

To a three neck flask containing **5b** (200mg, 0.72mmol) in toluene (4ml) under a nitrogen atmosphere at room temperature was added tetrakis(triphenylphosphine) palladium (105mg, 0.08mmol). After stirring for 10mins, Caesium carbonate (1.4g, 4.32mmol) was added followed by phenylboronic acid (96mg, 0.93mmol). The reaction was refluxed at 110°C for 3 hours. When cooled, the reaction was filtered through celite which was subsequently washed with ethyl acetate. The organic layer was then separated from the aqueous and dried over MgSO₄. The crude compound was concentration under reduced pressure and purified using column chromatography eluted with EtOAc to produce the pure compound as an off white solid. Yield 55mg, 23%.
¹H nmr (250MHz, CDCl₃); 7.50 (2H, d, *J* 7.2, CHₐᵣ), 7.45-7.29 (10H, m, CHₐᵣ), 7.07 (1H, d, *J* 7.8, CHₐᵣ), 5.84 (1H, s, CHO), 4.90 (1H, d, *J* 12.8, ArCHₐCH_{b}), 4.23 (1H, td, *J* 10.5, 2.5, OCHₐCH_{b}), 3.91 (1H, ddd, *J* 12.5, 5.7, 2.1, OCHₐCH_{b}), 3.79 (1H, d, *J* 12.7, ArCHₐCH_{b}), 2.90 (1H, ddd, *J* 14.2, 8.4, 2.1, NCHₐCH_{b}), 2.68 (1H, ddd, *J* 14.1, 5.6, 2.5, NCHₐCH_{b}), 2.52 (3H, s, NCH₃).

### 9-(3,5-dimethylisoxazol-4-yl)-5-methyl-1-phenyl-1,3,4,6-tetrahydro-5H-benz[f]-2,5-oxazocine (10a)

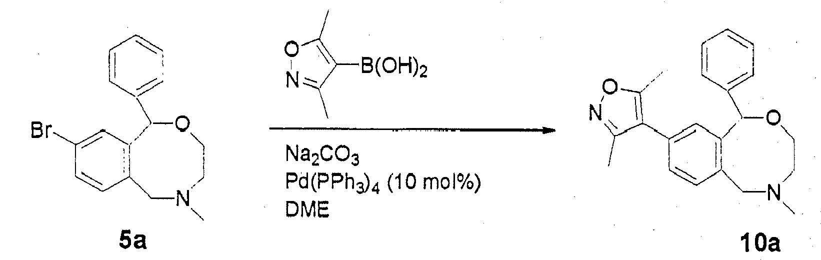

To a three neck flask containing **5a** (300mg, 0.91 mmol) in DME (4ml) under a nitrogen atmosphere at room temperature was added tetrakis(triphenylphosphine) palladium (105mg, 0.09mmol), After stirring for 10 mins, Na₂CO₃ (576mg, 5.44mmol) dissolved in water (2ml) was added followed by 3,5-dimethyl-4-isoxazolylboronic acid (166mg, 1.18mmol). The reaction was refluxed at 85°C for 2 hours. When cooled, the reaction was filtered through celite which was subsequently washed with ethyl acetate. The organic layer was then separated from the aqueous and dried over MgSO₄- Concentration under reduced pressure and column chromatography (10% MeOH in DCM) gave a brown oil. Yield 304mg, 97%.
¹H nmr (250MHz, CDCl₃); 7.28 (6H, m, CHₐᵣ), 7.13 (1H, dd, *J* 7.6, 1.5, CHₐᵣ), 6.85 (1H, d, *J* 1.1, CHₐᵣ), 5.84 (1H, S, CHO), 4.76 (1H, d, *J* 12.8, ArCHₐCH_{b}), 4.19 (1H, m, OCHₐCH_{b}), 3.90 (1H, m, OCHₐCH_{b}), 3.73 (1H, d, *J* 12.8, ArCHₐCH_{b}), 2,89 (1H, ddd, d, *J* 14.1, 7.9, 1.8, NCHₐCH_{b}), 2.68 (1H, ddd, *J* 14.1, 6.1, 2.2, NCHₐCH_{b}), 2.50 (3H, s, NCH₃), 2.26 (3H, s, CH₃), 2.12 (3H, s, CH₃).

### 8-(3,5-dimethylisoxazol-4-yl)-5-methyl-1-phenyl-1,3,4,6-tetrahydro-5H-benz[f]-2,5-oxazocine (10b)

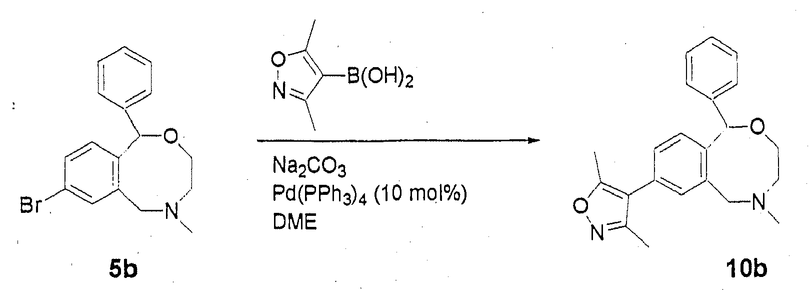

To a three neck flask containing **5b** (382mg, 1.15mmol) in DME (5ml) under a nitrogen atmosphere at room temperature was added tetrakis(triphenylphosphine) palladium (133mg, 0.12mmol). After stirring for 10 mins, Na₂CO₃ (734mg, 6,92mmol) dissolved in water (2ml) was added followed by 3,5-dimethyl-4-isoxazolylboronic acid (212mg, 1.5mmol). The reaction was refluxed at 85°C for 2 hours. When cooled, the reaction was filtered through celite which was subsequently washed with ethyl acetate. The organic layer was then separated from the aqueous and dried over MgSO4. Concentration under reduced pressure and column chromatography (10% MeOH in DCM) gave a brown oil. Yield 269mg, 67%.
¹H nmr (250MHz, CDCl₃); 7.30 (5H, m, CHₐᵣ), 7.08 (3H, m, CHₐᵣ), 5.82 (1H, s, CHO), 4.88 (1H, d, *J* 12.8, ArCHₐCH_{b}), 4.21 (1H, m, OCHₐCH_{b}), 3.89 (, ddd, J 12.6, 5.7, 2.2, OCHₐCH_{b} ), 3.72 (1H, d, *J* 13.0, ArCHₐCH_{b}), 2.86 (1H, ddd, d, *J* 14,2, 8.3, 2.2, NCHₐCH_{b}), 2.67 (1H, ddd, *J* 14.2, 5.7, 2.6, NCHₐCH_{b}), 2.47 (3H, s, NCH₃), 2.41 (3H, s, CH₃), 2.27 (3H, s, CH₃).

### 2-(5-methyl-1-phenyl-1,3,4,6-tetrahydro-5H-benz[f]-2,5-oxazocine-9-ethenyl)carboxamide (11a)

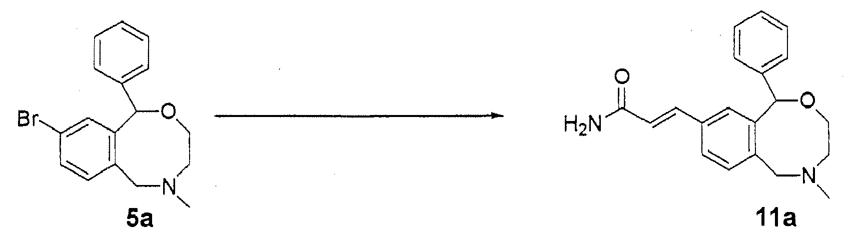

To a sample vial containing a solution of **5a** (245mg, 0.74mmol), in DMF (3ml) was added acrylamide (1 05mg, 1.47mmol), followed by Pd(OAc)₂ (17mg, 0.074mmol), P(*o*-tolyl)₃ (64mg, 0.21 mmol), NaOAc (73mg, 0.88mmol), LiCl (63mg, 1.47mmol), K₂CO₃ (122mg, 0.88mmol), and water (300µL) sequentially. The reaction vial was sealed and placed in a personal chemistry Microwave, Emrys optimiser at 140°C for 4 min. On cooling, the compound was purified by column chromatography, gradient elution of DCM; MeOH (5%, 10%, 15% and finally 20%). The compound was isolated as a colourless solid, (138mg, 58% yield).
¹H nmr (250MHz, CDCl₃); 7.50 (H, d, *J* 16.8, CHₐCONH), 7.46-7.21 (7H, m, CHₐᵣ), 7.12 (H, d, *J* 1.6, CHₐᵣ), 6.34 (H, d, *J* 16.8, CH_{b}CONH) 5.78 (3H, brs, NH₂,CHO), 4.83 (1H, d, *J* 13.8, ArCHₐCH_{b}), 4.20 (1H, ddd, *J* 13.5, 9.2, 2.9 OCHₐCH_{b}), 3.85 (1H, ddd, J 13.5, 5.9, 2.3, OCHₐCH_{b} ), 3.72 (1H, d, *J* 13.8, ArCHₐCH_{b}), 2.82 (1H, ddd, d, *J* 15.1, 8.9, 2.3, NCHₐCH_{b}), 2.64 (1H, ddd, *J* 15.4, 6.2, 2.9 NCHₐCH_{b}), 2.46 (3H, s, NCH₃).

### 2-(5-methyl-1-phenyl-1,3,4,6-tetrahydro-5H-benz[f]-2,5-oxazocine-8-ethenyl)carboxamide (11b)

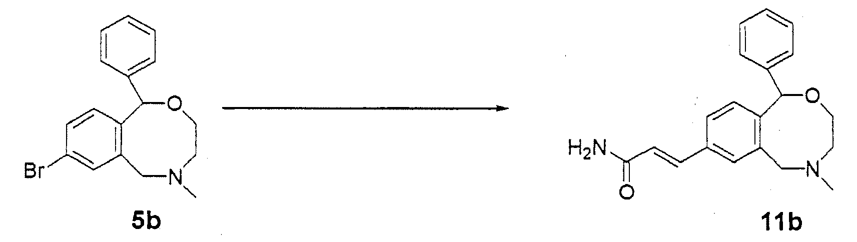

To a sample vial containing a solution of **5b** (247mg, 074mmol), in DMF (3ml) was added acrylamide (106mg, 1.47mmol), followed by Pd(OAc)₂ (17mg, 0.07mmol), P(*o*-tolyl)₃ (64mg, 0.21 mmol), NaOAc (73mg, 0.88mmol), LiCl (63mg, 1.47mmol), K₂CO₃ (122mg, 0.88mmol), and water (300µL) sequentially. The reaction vial was sealed and placed in a personal chemistry Microwave, Emrys optimiser at 140°C for 4 min. On cooling, the compound was purified by column chromatography, gradient elution of DCM; MeOH (5%, 10%, 15% and finally 20% to elute the product). The compound was isolated as a colourless solid, (51 mg, 21 % yield).
¹H nmr (250MHz, CDCl₃); 7.62 (H, d, *J* 15.6, CHₐCONH), 7.39-7.26 (7H, m, CHₐᵣ), 7.03 (H, d, *J* 8.1 CHₐᵣ), 6.46 (H, d, *J* 15.7, CH_{b}CONH) 5.78 (H, s, CHO), 4.90 (1H, d, *J* 12.7 ArCHₐCH_{b}), 4.25 (1H, td, *J* 10.6, 2.7, OCHₐCH_{b}), 3.87 (1H, ddd, J 12.6, 3.5, 2.5, OCHₐCH_{b}), 3.71 (1H, d, *J* 12.7, ArCHₐCH_{b}), 2.85 (1H, ddd, d, *J* 14.1, 8.6, 2.2, NCHₐCH_{b}), 2.64 (1H, ddd, *J* 14.1, 5.1, 2.5, NCHₐCH_{b}), 2.50 (3H, s, NCH₃).

### 2-(5-methyl-1-phenyl-1,3,4,6-tetrahydro-5H-benz[f]-2,5-oxazocine-9-ethyl)carboxamide (12a)

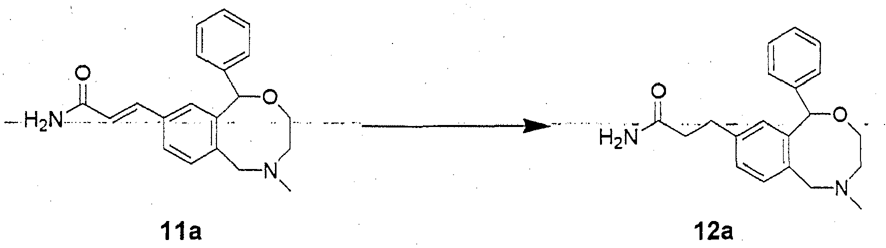

To a round bottom flask containing a solution of 2'-acrylamide-nefopam (130mg, 0,4mmol) in methanol (2ml) was added 20mg of 10% Pd on charcoal under N₂. On completion of addition, a balloon of H₂ was added and the reaction purged with H₂. The reaction mixture was then left at RT for 24 hours. Once the reaction was completed, the mixture was filtered through a plug of Celite and solvent evaporated. The crude reaction mixture was purified by column chromatography eluted with 10% MeOH:DCM (2 drops of TEA per 200ml). The compound was isolated as a yellow oil, (80mg, 62%).

¹H nmr (250MHz, MeOD); 7.30-7.04 (7H, m, CHₐᵣ), 6.91 (1H, s, CHₐᵣ), 5.75 (H, s, CHO), 4.87 (1H, d, *J* 13.6 ArCHₐCH_{b}), 4.16 (1H, td, *J* 9, 3, OCHₐCH_{b}), 3.83 (1H, ddd, J 12.6, 4.7, 2.4, OCHₐCH_{b}), 3.62 (1H, d, *J* 12.5, ArCHₐCH_{b}), 2.83 (3H, m, d, NCHₐCH_{b}, CH₂), 2.69-2.38 (6H, m, NCHₐCH_{b}, NCH₃, CH₂).

### 2-(5-methyl-1-phenyl-1,3,4,6-tetrahydro-5H-benz[f]-2,5-oxazocine-8-ethyl)carboxamide (12b)

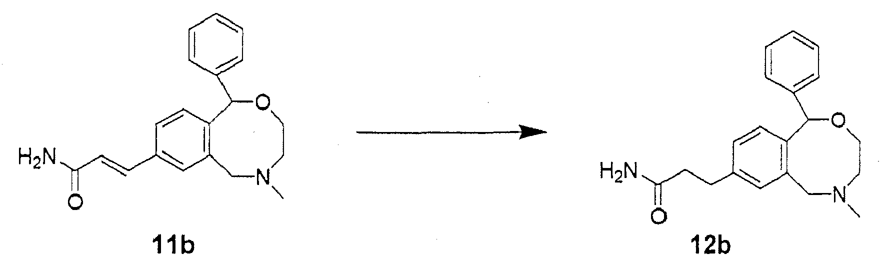

To a round bottom flask containing a solution of 3'-acrylamide-nefopam (96mg, 0.3mmol) in methanol (3ml) was added 15mg of 10% Pd on charcoal under N₂. On completion of addition, a balloon of H₂ was added and the reaction purged with H₂. The reaction mixture was then left at RT for 24 hours. Once the reaction was completed, the mixture was filtered through a plug of Celite and solvent evaporated. The crude reaction mixture was purified by column chromatography eluted with 10% MeOH:DCM (2 drops of TEA per 200ml). The compound was isolated as a yellow oil, (96mg, 100%).
¹H nmr (250MHz, MeOD); 7.34-7.23 (5H, m, CHₐᵣ), 7.07-7.02 (2H, m, CHₐᵣ), 6.92 (1H, d, *J* 8, CHₐᵣ, 5.77(H, s, CHO), 5.59 (2H, brs, NH₂), 4.71 (1H, d, *J* 12.8, ArCHₐCH_{b}), 4.16 (1H, ddd, *J* 12.5, 8.3, 2.8 OCHₐCH_{b}), 3.85 (1H, ddd, J 12.5, 5.8, 2.1, OCHₐCH_{b} ), 3.65 (1H, d, *J* 12.5, ArCHₐCH_{b}), 2.93 (2H, t, *J* 7.3, CH₂), 2.83 (1H, ddd, 14, 7.9, 2.2, NCHₐCH_{b}), 2.60 (H, ddd, *J* 14, 5.8, 2.45, NCHₐCH_{b}), 2.57-2.48 (5H, m, CH₂,NCH₃,CH₂).

### 5-methyl-1 -phenyl-1,3,4,6-tetrahydro-5H-benz[f]-2,5-oxazocine-9-methylamine (13a)

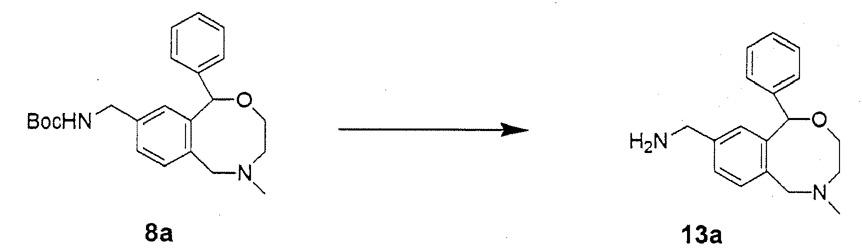

A dry round bottom flask was charged with **8a** (70mg, 0.18mmol), and TFA (1 ml). The reaction was stirred at RT under N₂ for 1 hour. Once the reaction was completed, the solution was diluted with water (10ml) and the mixture extracted three times with ether (10ml). The aqueous portion was base washed with 1 M NaOH until PH 14, the aqueous phase was then extracted three times with DCM (20ml). The organic phase was then dried over MgSO₄ and solvent removed under reduced pressure. The compound was obtained as a yellow oil (25mg, 60% yield).
¹H nmr (250MHz, CDCl₃); 7.31-7.18 (7H, m, CHₐᵣ), 6.92 (H, s, CHₐᵣ), 5.76 (H, s, CHO), 4.82 (1H, d, *J* 13, ArCHₐCH_{b}), 4.19 (1H, td, *J* 8, 3, OCHₐCH_{b}), 3.89-3.65 (4H, m, OCHₐCH_{b}, ArCHₐCH_{b}, CH₂), 2.82(1H, ddd, d, *J* 14, 8, 2 NCHₐCH_{b}), 2.60 (1H, ddd, *J* 14, 5, 2, NCHₐCH_{b}), 2.46 (3H, s, NCH₃), 1.25 (2H, brs, NH₂).

### 5-methyl-1-phenyl-1,3,4,6-tetrahydro-5H-benz[f]-2,5-oxazocine-8-methylamine (13b)

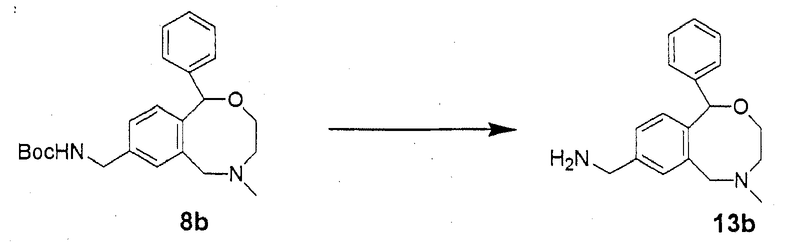

A dry round bottom flask was charged with **8b** (60mg, 0.16mmol), and TFA (1ml). The reaction was stirred at RT under N₂ for 1hour. Once the reaction was completed, the solution was diluted with water (10ml) and the mixture extracted three times with ether (10ml). The aqueous portion was base washed with 1 M NaOH until PH 14; the aqueous phase was then extracted three times with DCM (20ml). The organic phase was then dried over MgSO₄ and solvent removed under reduced pressure. The compound was obtained as yellow oil. (26mg, 60% yield).
¹H nmr (250MHz, CDCl₃); 7.31-7.10 (8H, m, CHₐᵣ), 6.97 (1H, d, *J* 7.6 CHₐᵣ), 5.79 (H, s, CHO), 4,91 (1H, d, *J* 12.5, ArCHₐCH_{b}), 4.17 (1H, ddd, *J* 12.8, 8.3, 2.8, OCHₐCH_{b}), 4.0 (2H, brs, NH₂), 3.89-3.83 (3H, m, OCHₐCH_{b}, CH₂), 3.67 1H, d, *J* 12.5, ArCHₐCH_{b}), 2.85 (1H, ddd, d, *J* 14, 8, 2 NCHₐCH_{b}), 2.65 (1H, ddd, *J* 14, 5.7, 2.4, NCHₐCH_{b}), 2.49 (3H, s, NCH₃).

### 2-(4-Methoxy)benzoyl-4-bromobenzoic acid and 2-(4-methoxy)benzoyl-5-bromobenzoic acid (14)

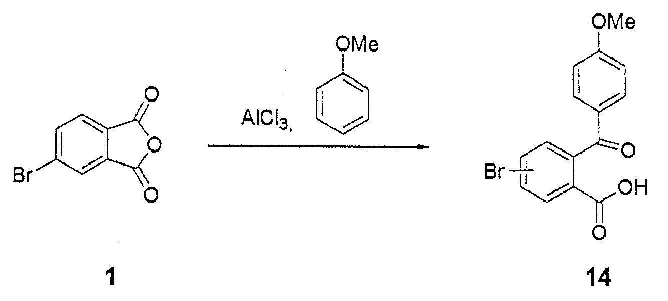

To a flask containing 4-bromophthalic anhydride (1) (25.65g, 0.11mol) was added finely crushed aluminium chloride (30.13g, 0.23mol). The solid mixture was further crushed and stirred using a spatula. To this mixture was added anisole (85,53g, 86ml, 0.79mol) which initiated the production of HCl gas. Once gas evolution ceased the reaction was heated at 80°C for 1.5 hours. The hot reaction mixture was poured into a solution of water : conc. hydrochloride acid (9:1, 600mL) and the aqueous layer extracted with ether (2 x 150mL). The organic layer was washed with water and then brine before being dried over magnesium sulphate, filtered and evaporated under reduced pressure to furnish the crude product. Overnight recrystallisation from a mixture of ether (200ml) and hexane (400ml) yielded **14**, after filtration, a white solid as a mixture of regio isomers. Yield 22.77g, 60%.
¹H nmr (250MHz, CDCl₃); 9.28 (2H, brs, 2xCO₂H), 8.19 (1H, d, *J* 1.8, CHₐᵣ), 7.92 (1H, d, *J* 8.4, CHₐᵣ), 7.78 (1H, d, *J* 1.8, CHₐᵣ), 7.74 (1H, d, *J* 1.8, CHₐᵣ), 7.69 (2H, d, *J* 2.0, CHₐᵣ), 7.65 (2H, d, *J* 1.8, CHₐᵣ), 7.48 (1H, d, *J* 1.8, CHₐᵣ), 7.22 (1H, d, *J* 8.1 CHₐᵣ), 6.91 (2H, d, *J* 2.5, CHₐᵣ), 6.88 (2H, d, J2.5, CHₐᵣ), 3.86 (6H, s, 2xCH₃).

### N-(2-hydroxyethyl)-N-methyl- 2-(4-methoxy)benzoyl-4-bromobenzamide (15a) and N-(2-hydroxyethyl)-N-methyl- 2-(4-methoxy)benzoyl-5-bromobenzamide (15b)

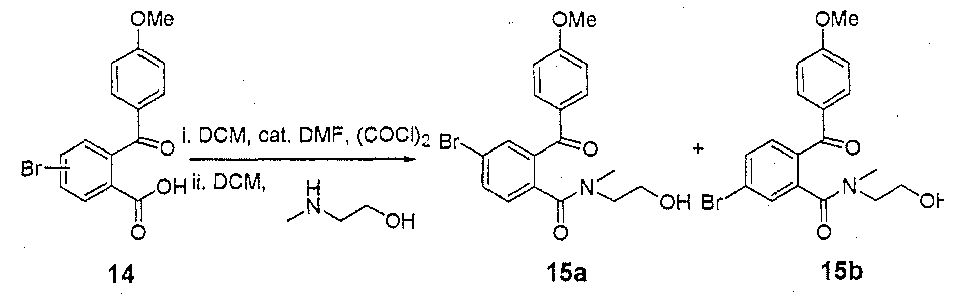

A solution of 2M oxalyl chloride (6.52mL, 74.73mmol, 1.1 equiv.) in dichloromethane was added dropwise to a suspension of the mixture of isomers **14** (22.77g, 67.94mmol) in dichloromethane (115mL) and catalytic *N*,*N-*dimethylformamide (5 drops) at room temperature under a nitrogen atmosphere. Gas evolution was rapid and as the reaction proceeded the solid dissolved in the dichloromethane. After 2.5 hours the solvent was removed under reduced pressure and the resulting solid co-evaporated with dichloromethane (2 x 100mL) to remove traces of excess oxalyl chloride, The crude acid chloride was dissolved in dichloromethane (115mL) and added dropwise to a solution of *N-*methylaminoethanol (6.0mL, 74.73mmol, 1.1 equiv.) and triethylamine, (10.42mL, 74.73mmol, 1.1 equiv) in dichloromethane (115mL) cooled to 0°C in an ice bath. The resulting solution was stirred at room temperature for 3 hours, quenched with saturated aqueous sodium dicarbonate (100mL) and separated. The aqueous layer was extracted with dichloromethane (100mL) and the combined organic fractions washed with brine (50mL), dried over magnesium sulfate and filtered. The solvent was removed under reduced pressure and the crude product purified by column chromatography, eluting with ethyl acetate : hexane (4:1), followed by ethyl acetate (100%) as product eluted. Compound **15a** was furnished as a yellow semi solid. Yield 5.00g, 19%. The product exists are a mixture of rotomers in a 3:2 ratio.
¹H nmr (250MHz, CDCl₃); 7.81 (2H, d, *J* 7.5, CHₐᵣ), 7.71-7.53 (2H, m, CHₐᵣ), 7.31 (1H, m, CHₐᵣ), 6.95 (2H, d, J7.6, CHₐᵣ), 3.88 (3H, s, OCH₃), 3.80 (2H, t, *J* 5.1, CH₂OH), 3.72 (1H, m OH), 3.57 (2H, t, *J* 4.9, NCH₂), 3.06 (1.2H, s, NCH₃) 2.96 (1.8H, s, NCH₃).

Compound **15b** was furnished as a red semi solid. Yield 5.00g, 19%. The products exist as a mixture of rotomers in a 3:2 ratio.
¹H nmr (250MHz, CDCl₃); 7.77 (2H, dd, J 9.0, 2.4, CHₐᵣ), 7.59-7.53 (2H, m, CHₐᵣ), 7.42-7.31 (1H, m, CHₐᵣ), 6.91 (2H, dd, *J* 9.0, 2.4, CHₐᵣ), 3.85 (3H, s, OCH₃), 3.79 (2H, t, *J* 5.1, CH₂OH), 3.69 (1H, brs OH), 3.56 (2H, t, *J* 5.1, NCH₂), 3.03 (1.2H, s, NCH₃) 2.95 (1.8H, s, NCH₃).

### N-(2-hydroxyethyl)-N-methyl-4-bromo-2-[1-hydroxy-1-(4-methoxyphenyl)] methylbenzylamine (16a)

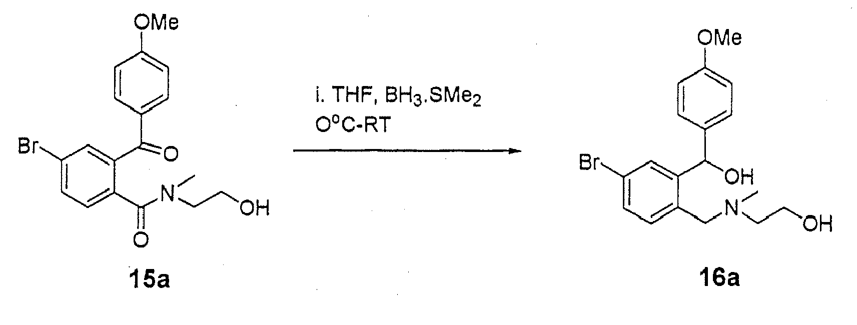

Amide **15a** (1.56g, 3.99mmol) was dissolved in tetrahydrofuran (10mL) and cooled to 0°C in an ice bath. A 2M solution of borane dimethylsulfide complex (8.78mL, 17,55mmol, 4.4 equiv.) was added dropwise and the resulting solution stirred at room temperature for 17 hours. The reaction was carefully quenched with 2M hydrochloric acid solution (5mL) and the resulting solution stirred at room temperature for 2 hours. Tetrahydrofuran was removed under reduced pressure and remaining solution diluted with water (10mL) and extracted with diethyl ether (2 x 50mL). The aqueous layer was basified with 3.75M sodium hydroxide solution and extracted with ethyl acetate (2 x 50mL). The combined ethyl acetate extracts were dried over magnesium sulfate, filtered and evaporated under reduced pressure to furnish the desired product 16a as a white foam. Yield 754mg, 50%.
¹H nmr (250MHz, CDCl₃); 7.39-7.24 (4H, m, CHₐᵣ), 7.07-6.98 (1H, m, CHₐᵣ), 6.89-6.79 (2H, m, CHₐᵣ), 5.77 (1H, s, CHOH), 3.80 (3H, s, OCH₃), 3.67 (2H, m, OCH₂), 3.41 (1H, d, *J*, 12.4, ArCHₐH_{b}N), 3.28 (1H, d, *J*, 12,7, ArCHₐH_{b}N), 2.57-2.50 (2H, m, NCH₂), 2.17 (3H, s, NCH₃).

### N-(2-hydroxyethyl)-N-methyl-5-bromo-2-[1-hydroxy-1-(4-methoxyphenyl)] methylbenzylamine (16b)

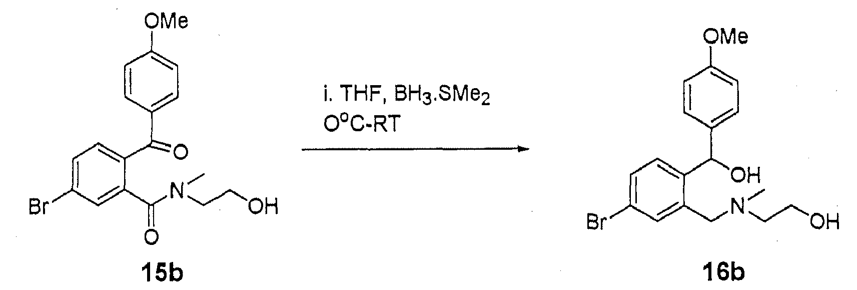

Amide **15b** (1.54g, 3.93mmol) was dissolved in tetrahydrofuran (10mL) and cooled to 0°C in an ice bath. A 2M solution of borane dimethylsulfide complex (8.60mL, 17.28mmol, 4.4 equiv.) was added dropwise and the resulting solution stirred at room temperature for 17 hours. The reaction was carefully quenched with 2M hydrochloric acid solution (5mL) and the resulting solution stirred at room temperature for 2 hours. Tetrahydrofuran was removed under reduced pressure and remaining solution diluted with water (10mL) and extracted with diethyl ether (2 x 50mL). The aqueous layer was basified with 3.75M sodium hydroxide solution and extracted with ethyl acetate (2 x 50mL). The combined ethyl acetate extracts were dried over magnesium sulfate, filtered and evaporated under reduced pressure to furnish the desired product **16b** as a white foam. Yield 606mg, 40%.
¹H nmr (250MHz, CDCl₃); 7.45-7.33 (2H, m, CHₐᵣ), 7.24 (2H, d, *J* 8.4, CHₐᵣ), 7.03-6.95 (1H, m, CHₐᵣ), 6.86 (2H, d, *J* 8.7, CHₐᵣ), 5.79 (1H, s, CHOH), 3.79 (3H, s, OCH₃), 3.65 (2H, m, OCH₂), 3.41 (1H, d, *J,* 12.5, ArCHₐH_{b}N), 3.28 (1H, d, *J,* 12.5, ArCHₐH_{b}N), 2.56-2.50 (2H, m, NCH₂), 2.18 (3H, s, NCH₃).

### 9-Bromo-5-methyl-1-(4-methoxy)phenyl-1,3,4,6-tetrahydro-5H-benz[f]-2,5-oxazocine (17a)

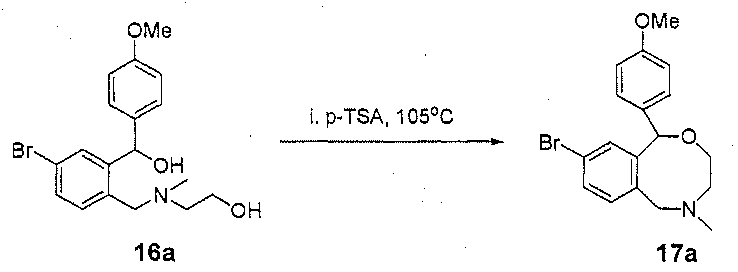

Diol **16a** (754mg, 1.99mmol) was dissolved in toluene (10mL) and *para*toluenesulfonic acid monohydrate (568mg, 2.98mmol, 1.5equiv.) added. The toluene was removed under reduced pressure and the resulting oil heated at 105°C for 2 hours. On cooling the oil was suspended in water (10mL) and basified with 3.75M sodium hydroxide solution. The aqueous layer was extracted with ethyl acetate (2 x 25mL), dried over magnesium sulfate, filtered and evaporated under reduced pressure to furnish the crude product which was purified by chromatography eluting with 10% methanol in ethyl acetate. Fractions containing product were combined and evaporated under reduced pressure to furnish **17a,** Yield 340mg, 47%.
¹H nmr (250MHz, CDCl₃); 7.34 (1H, dd, J 8.1, 1.8, CHₐᵣ), 7.16 (2H, d, *J* 8.7, CHₐᵣ), 7.12-7.06 (2H, m, CHₐᵣ), 6.85 (2H, d, J 8.6, CHₐᵣ), 5.66 (1H, s, CHOH), 4.83 (1H, d, *J* 12.8, ArCHₐH_{b}), 4.15 (1H, m, OCHₐH_{b}), 3.83 (1H, OCHₐH_{b}), 3.78 (3H, s, OCH₃), 3.60 (1H, d, *J* 12.8, ArCHₐH_{b}), 2.77 (1H, m NCHₐH_{b}), 2.59 (1H, ddd, *J* 2.6, 5.5, 14.2, NCHₐH_{b}), 2.43 (3H, s, CH₃).

### 8-Bromo-5-methyl-1-(4-methoxy)phenyl-1,3,4,6-tetrahydro-5H-benz[f]-2,5-oxazocine (17b)

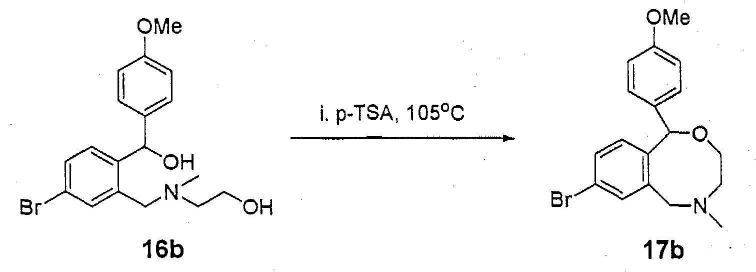

Diol **16b** (200mg, 0.53mmol) was dissolved in toluene (5mL) and *para*toluenesulfonic acid monohydrate (150mg, 0.79mmol, 1.5equiv.) added. The toluene was removed under reduced pressure and the resulting oil heated at 105°C for 2 hours. On cooling the oil was suspended in water (10mL) and basified with 3.75M sodium hydroxide solution. The aqueous layer was extracted with ethyl acetate (2 x 25mL), dried over magnesium sulfate, filtered and evaporated under reduced pressure to furnish the crude product which was purified by chromatography eluting with 10% methanol in ethyl acetate. Fractions containing product were combined and evaporated under reduced pressure to furnish **17b** as a clear oil. Yield 127mg, 67%.
¹H nmr (250MHz, CDCl₃); 7.36 (1H, d, *J* 1.5, CHₐᵣ), 7.29 (1H, dd, *J* 8.4, 1.8, CHₐᵣ), 7.15 (2H, d, *J* 8.7, CHₐᵣ), 6.85 (1H, m, CHₐᵣ), 6.83 (2H, d, *J* 8.6, CHₐᵣ), 5.68 (1H, s, CHOH), 4.81 (1H, d, *J* 12.8, ArCHₐH_{b}), 4.13 (1H, m, OCHₐH_{b}), 3.83 (1H, OCHₐH_{b}), 3.76 (3H, s, OCH₃), 3.58 (1H, d, *J* 12.8, ArCHₐH_{b}), 2.77 (1H, m NCHₐH_{b}), 2.61 (1H, ddd, *J* 2.5, 5.7, 14.1, NCHₐH_{b}), 2.45 (3H, S, CH₃).

### 2-(3-Methoxy)benzoyl-4-bromobenzoic acid and 2-(3-methoxy)benzoyl-5-bromobenzoic acid (18)

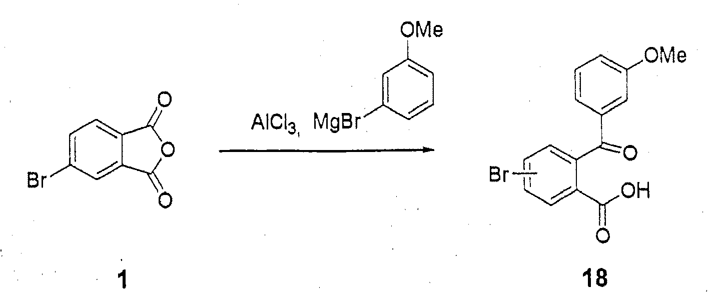

Magnesium (2,45g, 0.1 mol) was suspended in anhydrous ether (250ml) under a nitrogen atmosphere at room temperature. 3-Bromoanisole (18.75g, 0.1 mol) and iodine (cat.) were added to form an initial red solution which became colourless after a few minutes. The reaction was allowed to stir overnight and a pale yellow precipitate formed, The precipitate was added, via a dropping funnel, to a stirred solution of 4-bromophthalic anhydride (**1**) (25g, 0.1 mol) in toluene (150ml) and ether (30ml) under a nitrogen atmosphere. The subsequent reflux was maintained for 24 hours, cooled and quenched with saturated aqueous NH₄Cl. Thw aqueous layer was extracted with diethyl ether (3 x 200ml), dried over MgSO₄, filtered and concentrated under reduced pressure to give the desired mixture of regioisomers (30.1g, 82% yield).
¹H nmr (250MHz, CD₃OD); 8.19 (H, s, CHₐᵣ), 7.92 (H, d, CHₐᵣ), 7.74 (2H, dd, J23.5, 8.7, CHₐᵣ), 7.51 (H. s, CHₐᵣ), 7.36-7.09 (9H, m, CHₐᵣ), 6.95-6.85 (H, m, CHₐᵣ), 6.52-6.40 (H, m, CHₐᵣ), 2.35 (6H, s, OCH₃),

### N-(2-hydroxyethyl)-N-methyl- 2-(3-methoxy)benzoyl-4-bromobenzamide (19a) and N-(2-hydroxyethyl)-N-methyl- 2-(3-methoxy)benzoyl-5-bromobenzamide (19b)

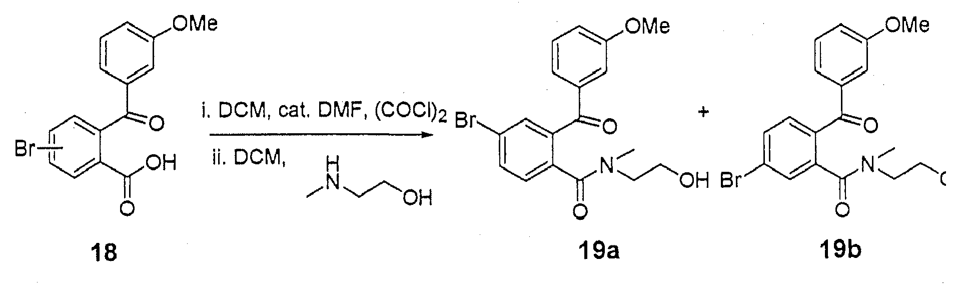

A solution of 2M oxalyl chloride (8.65mL, 0.1mol) in dichloromethane was added dropwise to a suspension of the mixture of isomers **18** (30.0g, 0.09mol) in dichloromethane (200ml) and catalytic *N*,*N*-dimethylformamide (1 drop) at room temperature under a nitrogen atmosphere. Gas evolution was rapid and as the reaction proceeded the solid dissolved in the dichloromethane. After 2.5 hours the solvent was removed under reduced pressure and the resulting solid co-evaporated with dichloromethane to remove traces of excess oxalyl chloride. The crude acid chloride was dissolved in dichloromethane (200mL) and added dropwise to a solution of *N*-methylaminoethanol (6mL, 0.1 mol.) and triethyamine (14mL) in dichloromethane (200mL) cooled to 0°C in an ice bath. The resulting solution was stirred at room temperature overnight, quenched with saturated aqueous ammonium chloride and separated. The organic layer was washed with water (2 x 400mL), dried over magnesium sulfate and filtered. The solvent was removed under reduced pressure and the crude product purified by chromatography, eluting with hexane : ethyl acetate, (1:1) to yield 19a (8.88g, 25% yield) and **19b** (6,13g, 18% yield) as viscous oils.
**19a**:
¹H nmr (250MHz, CDCl₃); 7.61-7.56 (3H, m, CHₐᵣ), 7.49-7.44 (H, m, CHₐᵣ), 7.39-7.26 (8H, m, CHₐᵣ), 7.17-7.13 (2H, m, CHₐᵣ), 3.86-3.83 (9H, m, OCH₃, CH₂), 3.61 (4H, t, *J* 5.3, CH₂), 3.09 (3H, s, NCH₃), 2.99 (3H, s, NCH₃), 1.41 (2H, brs, OH).

### N-(2-hydroxyethyl)-N-methyl-4-bromo-2-[1-hydroxy-1-(3-methoxyphenyl)] methylbenzylamine (20a)

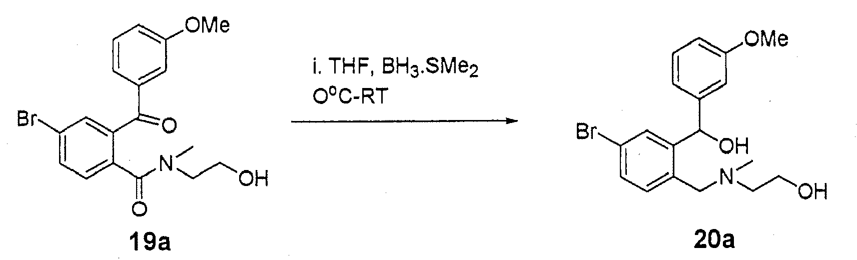

Amide **19a** (700mg, 1.78mmol) was dissolved in tetrahydrofuran (3mL) and cooled to 0°C in an ice bath. A 2M solution of borane dimethylsulfide complex (3.6mL, 7.12mmol, 4.4 equiv.) was added dropwise and the resulting solution stirred at room temperature for 17 hours. The reaction was carefully quenched with 1 M hydrochloric acid solution and the resulting solution stirred overnight. Tetrahydrofuran was removed under reduced pressure and the remaining solution washed with diethyl ether until all by-products had been removed. The aqueous layer was basified with NaOH (2M) and extracted using ethyl acetate (30ml). After washing with NaOH (2x50ml) and water (50ml), the ethyl acetate was dried over MgSO₄, filtered and concentrated under reduced pressure to furnish the desired product **20a** as a viscous oil. Yield 400mg, 59%.
¹H nmr (250MHz, CDCl₃); 7.37-7.31 (2H, m, CHₐᵣ), 7.21 (1H, t, *J* 8.5, CHₐᵣ), 7.05-6.98 (2H, m, CHₐᵣ), 6.85-6.77 (2H, m, CHₐᵣ), 5.75 (1H, s, CHOH), 3.77 (3H, s, OCH₃), 3.65-3.61 (2H, m, OCH₂), 3.29 (2H, dd, *J*, 25.3,13.5, ArCHₐH_{b}N), 2.50 (2H, dd, *J* 10.9, 5.4, NCH₂), 2.02 (3H, s, NCH₃).

### N-(2-hydroxyethyl)-N-methyl-5-bromo-2-[1-hydroxy-1-(4-methoxyphenyl)] methylbenzylamine (20b)

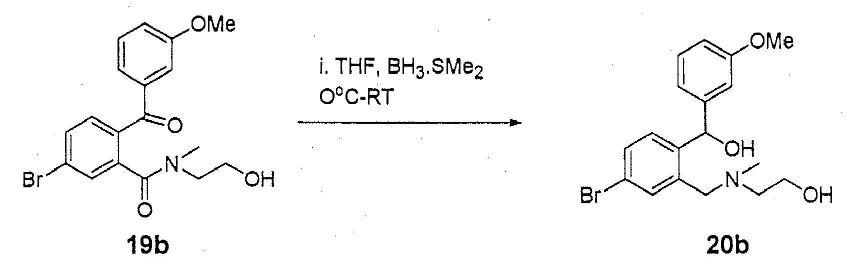

Amide **19b** (550mg, 1.40mmol) was dissolved in tetrahydrofuran (3mL) and cooled to 0°C in an ice bath. A 2M solution of borane dimethylsulfide complex (2.8mL, 5.60mmol, 4.4 equiv.) was added dropwise and the resulting solution stirred at room temperature for 17 hours. The reaction was carefully quenched with 1 M hydrochloric acid solution and the resulting solution stirred overnight. Tetrahydrofuran was removed under reduced pressure and the remaining solution washed with diethyl ether until all by-products had been removed. The aqueous layer was basified with NaOH (2M) and extracted using ethyl acetate (30ml). After washing with NaOH (2x5Oml) and water (50ml), the ethyl acetate was dried over MgSO₄, filtered and concentrated under reduced pressure to furnish the desired product 20b as a viscous oil. Yield 300mg, 56%.
¹H nmr (250MHz, CDCl₃); 7.40-7.35 (3H, m, CHₐᵣ), 7.26-7.20 (2H, m, CHₐᵣ). 7.07 (1H, d, *J* 8,6, CHₐᵣ), 5.80 (1H, s, CHOH), 3.79 (3H, s, OCH₃) 3.71-3.66 (2H, m, OCH₂), 3.36 (2H, dd, *J,* 19.6, 12.5, ArCH₂N), 2.62-2.49 (2H, m, NCH₂), 2.04 (3H, s, NCH₃).

### 9-Bromo-5-methyl-1-(3-methoxy)phenyl-1,3,4,6-tetrahydro-5H-benz[f]-2,5-oxazocine (21a)

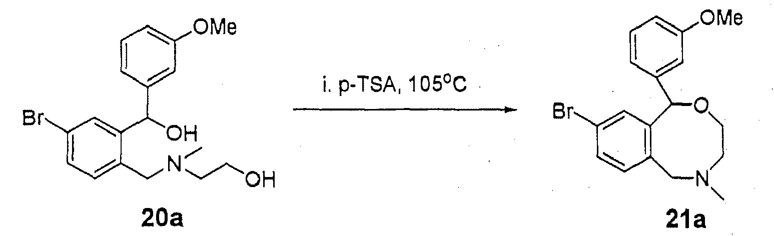

Diol **20a** (400mg, 1.05mmol) was dissolved in toluene (2mL) and *para*toluenesulfonic acid monohydrate (300mg, 1.58mmol, 1.5equiv.) added. The toluene was removed under reduced pressure and the resulting oil heated at 105°C for 4 hours. On cooling the oil was suspended in water (100mL) and basified with 3.75M sodium hydroxide solution. The aqueous layer was extracted with ethyl acetate, dried over magnesium sulfate, filtered and evaporated under reduced pressure to furnish the crude product. Purification by column chorography (10% methanol in ethyl acetate) furnished **21a** as yellow solid. Yield 71 mg, 19%.
¹H nmr (250MHz, CDCl₃); 7.39-7.05 (5H, m, CHₐᵣ), 6.84-6.79 (3H, m, CHₐᵣ), 5.66 (1H, s, CH), 4.78 (1H, d, *J* 13.8, ArCHₐH_{b}), 4.22-4.13 (1H, m, OCHₐH_{b}), 3.84-3.83 (1H, m, OCHₐH_{b}), 3.77 (3H, s, OCH₃), 3.64 (1H, d, *J* 13.7, ArCHₐH_{b}), 2.82-2.74 (1H, m NCHₐH_{b}), 2.64-2.57 (1H, m, CHₐH_{b}), 2.42 (3H, s, CH₃).

### 8-Bromo-5-methyl-1-(3-methoxy)phenyl-1,3,4,6-tetrahydro-5H-benz[f]-2,5-oxazocine (21b)

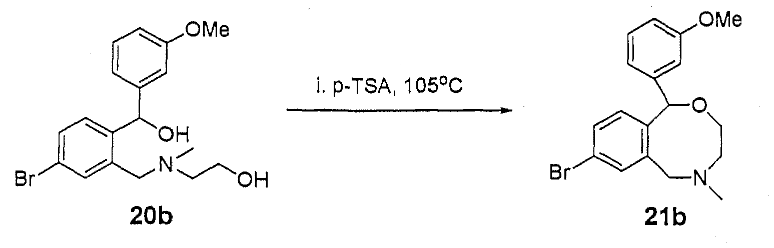

Diol **20b** (300mg, 0,79mmol) was dissolved in toluene (2mL) and *para*toluenesulfonic acid monohydrate (228mg, 1.2mmol, 1.5equiv.) added, The toluene was removed under reduced pressure and the resulting oil heated at 105°C for 4 hours. On cooling the oil was dissolved in 3.75M sodium hydroxide solution and extracted with ethyl acetate, dried over magnesium sulfate, filtered and evaporated under reduced pressure to furnish **21b** as a brown oil. Yield 75mg, 26%. No further purification was performed.
¹H nmr (250MHz, CDCl₃); 7.37-719 (3H, m, CHₐᵣ), 6.89-6.78 (4H, m, CHₐᵣ), 5.70((1H, s, CH), 4.75 (1H, d, *J* 14, ArCHₐH_{b}), 4.20-4.10 (1H, m, OCHₐH_{b}), 3.86-3.77 (4H, m, ArCHₐH_{b}), OCH₃), 3.60 (1H, d, *J* 14, ArCHₐH_{b}), 2.80(1H, ddd, *J*2.1, 8.5, 15.3, NCHₐH_{b}), 2.62 (1H, ddd, *J* 2.7, 6.4, 15.3, NCHₐH_{b}), 2.44 (3H, s, CH₃).

### 9-Cyano-5-methyl-1-(3-methoxy)phenyl-1,3,4,6-tetrahydro-5H-benz[f]-2,5-oxazocine (22a)

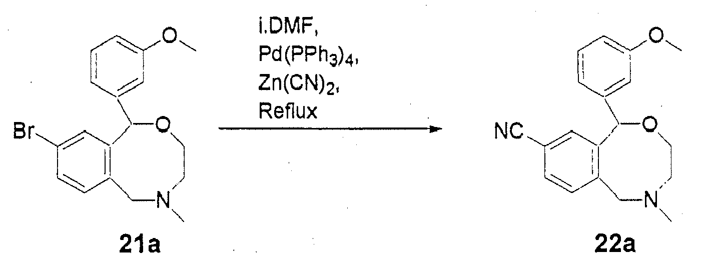

Bromo-nefopam analogue **21a** (503mg, 1.39mmol), Zn(CN)₂ (245mg, 2.09mmol), and Pd(PPh₃)₄ (241 mg, 0.21mmol), were dissolved in degassed anhydrous DMF (10mL) under a N₂ atmosphere. The mixture was refluxed under N₂ for 24 hours, The mixture was allowed to cool to room temperature, filtered through celite and washed through with DCM (50ml). The filtrate was then quenched with water (10ml) and solvent extracted. The organic extract was dried over MgSO₄, filtered and solvent removed under reduced pressure. The crude product was purified by dry flash chromatography eluting with 5%-15% MeOH in DCM. Fractions containing the product were combined and evaporated under reduced pressure to produce **22a** as pale brown oil. Yield 143mg, 33%,
¹H nmr (250MHz, CDCl₃); 7.51 (1H, dd, *J* 1.4, 7.8, CHₐᵣ), 7.32-7.23 (4H, m, CHₐᵣ), 6.84-6.81 (3H, m, CHₐᵣ), 5.76 (1H, s, CHO), 4.85 (1H, d, *J* 12.8, ArCHₐH_{b}), 4.18-4.15 (1H, m, OCHₐH_{b}), 3.86-3.81 (1H, m, OCHₐH_{b}). 3.79 (3H, s, OCH₃), 3.71 (1H, d, *J* 12.8, ArCHₐH_{b}), 2.78 (1H, ddd, *J* 2.3, 8.2, 14.3, NCHₐH_{b}), 2.63 (1H, ddd, *J* 2.8, 5.6, 14.3, NCHₐH_{b}), 2.44 (3H, s, NCH₃).

### 8-Cyano-5-methyl-1-(3-methoxy)phenyl-1,3,4,6-tetrahydro-5H-benz[f]-2,5-oxazocine (22b)

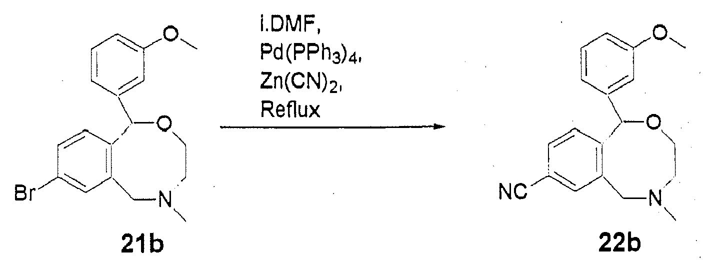

Bromo-nefopam analogue **21b** (223mg, 0.62mmol), Zn(CN)₂ (109mg, 0.93mmol), and Pd(PPh₃)₄ (107mg, 0.09mmol), were dissolved in degassed anhydrous DMF (5mL) under a N₂ atmosphere. The mixture was refluxed under N₂ for 24 hours. The mixture was allowed to cool to room temperature, filtered through celite and washed through with DCM (50ml). The filtrate was then quenched with water (10ml) and solvent extracted. The organic extract was dried over MgSO₄, filtered and solvent removed under reduced pressure. The crude product was purified by dry flash chromatography eluting with 5%-15% MeOH in DCM. Fractions containing the product were combined and evaporated under reduced pressure to produce **22b** as pale brown solid. Yield 94mg, 50%.
¹H nmr (250MHz, CDCl₃); 7.50 (1H, s, CHₐᵣ), 7.46 (1H, dd, *J* 1.7, 8.1, CHₐᵣ), 7.26-7.21 (1H, m, CHₐᵣ), 7.12 (1H, d, *J* 7.9, CHₐᵣ), 6.83-6.80 (3H, m, CHₐᵣ), 5.74 (1H, s, CHO), 4.94 (1H, d, *J* 13.0, ArCHₐH_{b}), 4.28-4.19 (1H, m, OCHₐH_{b}), 3.87-3.79 (1H, m, OCHₐH_{b}), 3.77 (3H, s, OCH₃), 3.65 (1H, d, *J* 13.0, ArCHₐH_{b}), 2.75 (1H, ddd, *J* 2.3, 8.3, 14.3, NCH₃H_{b}), 2.63 (1H, ddd, *J* 3.0, 5.4, 14.3, NCHₐH_{b}), 2.45 (3H, s, NCH₃).

### 9-Methoxy-5-methyl-1-phenyl-1,3,4,6-tetrahydro-5H-benz[f]-2,5-oxazocine (23a)

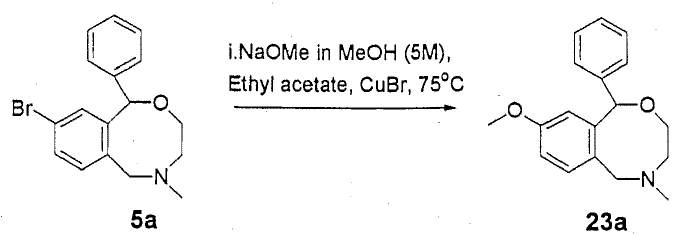

Ethyl acetate (0.1 ml) was added to a stirred 5M NaOMe methanol solution (1 ml) under an N₂ atmosphere at room temperature. Bromo-nefopam analogue **5a** (210mg, 0.58mmol) in MeOH (1ml) was then added followed by CuBr (17mg, 0.12mmol). The mixture was stirred at 75°C overnight, cooled to room temperature and quenched using water (5ml). The organic layer was separated and the aqueous was washed with ethyl acetate (2x10ml). The combined organics were dried over MgSO₄, filtered and concentrated under reduced pressure. The crude product was purified by dry flash chromatography eluting with 10% MeOH in DCM. Fractions containing the product were combined and evaporated under reduced pressure to produce **23a** as an orange oil. Yield 131 mg, 73%.
¹H nmr (250MHz, CDCl₃); 7.31-7.24 (5H, m, CHₐᵣ), 7.16 (1H, d, *J* 8.4, CHₐᵣ), 6.77 (1H, dd, *J* 2.7, 8.3, CHₐᵣ), 6.53 (1H, d, *J* 2.6, CHₐᵣ), 5.72 (1H, s, CHO), 4.74 (1H, d, *J* 12.8, ArCHₐH_{b}), 4.23-4.15 (1H, m, OCHₐH_{b}), 3.86 (1H, ddd, J2.3, 5.6, 12.6, OCHₐH_{b}), 3.71 (3H, s, OCH₃), 3.68 (1H, d, *J* 12.8, ArCHₐH_{b}), 2.84 (1H, ddd, *J* 2.3, 8.5, 14.2, NCHₐH_{b}), 2.62 (1H, ddd, *J* 2.7, 5.6, 14.3, NCHₐH_{b}), 2.45 (3H, s, NCH₃).

### 8-Methoxy-5-methyl-1-phenyl-1,3,4,6-tetrahydro-5H-benz[f]-2,5-oxazocine (23b)

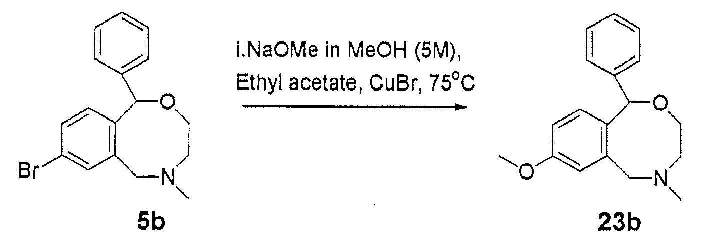

Ethyl acetate (0.1 ml) was added to a stirred 5M NaOMe methanol solution (1ml) under an N₂ atmosphere at room temperature. Bromo-nefopam analogue 5b (215mg, 0.60mmol) in MeOH (1ml) was then added followed by CuBr(17mg, 0.12mmol). The mixture was stirred at 75°C overnight, cooled to room temperature and quenched using water (5ml). The organic layer was separated and the aqueous was washed with ethyl acetate (2x10ml). The combined organics were dried over MgSO₄, filtered and concentrated under reduced pressure. The crude product was purified by dry flash chromatography eluting with 10% MeOH in DCM. Fractions containing the product were combined and evaporated under reduced pressure to produce **23b** as an orange oil. Yield 161 mg, 88%.
¹H nmr (250MHz, CDCl₃); 7.35-7.20 (5H, m, CHₐᵣ), 6.89 (1H, d, *J* 8.3, CHₐᵣ), 6.76 (1H, s, CHₐᵣ), 6.72 (1H, m, CHₐᵣ), 5.80 (1H, s, CHO), 4.64 (1H, d, *J* 12.8, ArCHₐH_{b}), 4.11 (1H, ddd, *J* 2.5, 8.0, 12.7, OCHₐH_{b}), 3.87 (1H, ddd, *J* 2.2, 6.0, 12.7, OCHₐH_{b}), 3.80 (3H, s, OCH₃), 3.70 (1H, d, *J* 12.8, ArCHₐH_{b}), 2.87 (1H, ddd, *J* 2.0, 8.0, 14.2, NCHₐH_{b}), 2.66 (1H, ddd, *J* 2.4, 6.1, 14.2, NCHₐH_{b}), 2.48 (3H, s, NCH₃).

### 9-Methoxy-5-methyl-1-(3-methoxy)phenyl-1,3,4,6-tetrahydro-5H-benz[f]-2,5-oxazocine (24a)

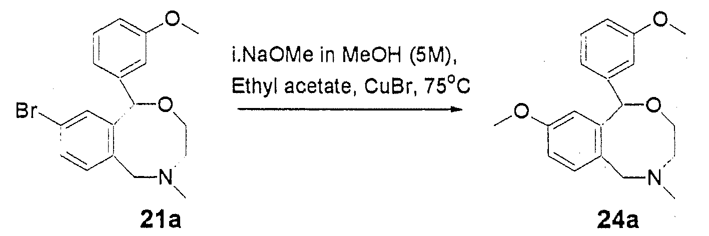

Ethyl acetate (0,1 ml) was added to a stirred 5M NaOMe methanol solution (1ml) under an N₂ atmosphere at room temperature. Bromo-nefopam analogue **21 a** (1 73mg, 0.48mmol) in MeOH (1ml) was then added followed by CuBr (14mg, 0.10mmol). The mixture was stirred at 75°C overnight, cooled to room temperature and quenched using water (5ml). The organic layer was separated and the aqueous was washed with ethyl acetate (2x10ml), The combined organics were dried over MgSO₄, filtered and concentrated under reduced pressure. The crude product was purified by dry flash chromatography eluting with 10% MeOH in DCM. Fractions containing the product were combined and evaporated under reduced pressure to produce **24a** as an orange oil. Yield 34mg, 22%.
¹H nmr (250MHz, CDCl₃); 7.20 (1H, d, *J* 8.2, CHₐᵣ), 7.15 (1H, s, CHₐᵣ), 6.87-6.84 (3H, m, CHₐᵣ), 6.77 (1H, dd, *J* 2.8, 8.3, CHₐᵣ), 6.54 (1H, d, *J* 2.6, CHₐᵣ), 5.68 (1H, s, CHO), 4.75 (1H, d, J 12.8, ArCHₐH_{b}), 4.24-4.15 (1H, m, OCHₐH_{b}). 3.85 (1H, ddd, *J* 2.4, 5.3, 12.7, OCHₐH_{b}), 3.76 (3H, s, OCH₃), 3.71 (1H, d, *J* 12.8, ArCHₐH_{b}), 3.70 (3H, s, OCH₃), 2.85 (1H, ddd, *J* 2.1, 8.7, 14.1, NCHₐH_{b}), 2.64 (1H, ddd, *J* 2.7, 5.3, 14.3, NCHₐH_{b}), 2.47 (3H, s, NCH₃).

### 8-Methoxy-5-methyl-1-(3-methoxy)phenyl-1,3,4,6-tetrahydro-5H-benz[f]-2,5-oxazocine (24b)

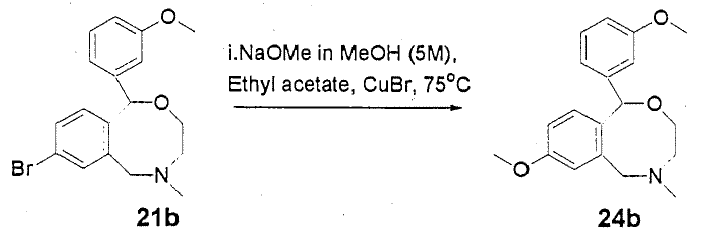

Ethyl acetate (0.1 ml) was added to a stirred 5M NaOMe methanol solution (1ml) under an N₂ atmosphere at room temperature. Bromo-nefopam analogue **21b** (168mg, 0,47mmol) in MeOH (1 ml) was then added followed by CuBr (13mg, 0.09mmol). The mixture was stirred at 75°C overnight, cooled to room temperature and quenched using water (5ml). The organic layer was separated and the aqueous was washed with ethyl acetate (2x10ml). The combined organics were dried over MgSO₄ filtered and concentrated under reduced pressure. The crude product was purified by dry flash chromatography eluting with 10% MeOH in DCM. Fractions containing the product were combined and evaporated under reduced pressure to produce **24b** as an orange oil. Yield 77mg, 50%.
¹H nmr (250MHz, CDCl₃); 7.22 (1H, t, *J* 8.2, CHₐᵣ), 6.92-6.71 (6H, m, CHₐᵣ), 5.78 (1H, s, CHO), 4.60 (1H, d, *J* 12.7, ArCHₐH_{b}), 4.09 (1H, ddd, *J* 2.4, 7.9, 12.7, OCHₐH_{b}), 3.90-3.82 (1H, m, OCHₐH_{b}), 3.80 (3H, s, OCH₃), 3.77 (3H, s, OCH₃), 3.70 (1H, d, *J* 12,8, ArCHₐH_{b}), 2.85 (1H, ddd, *J* 1.9, 7.9, 14.2, NCHₐH_{b}), 2.65 (1H, ddd, *J* 2.5, 6.1, 14.2, NCHₐH_{b}), 2.47 (3H, s, NCH₃).

### Cyclopropylboronic Acid (25)

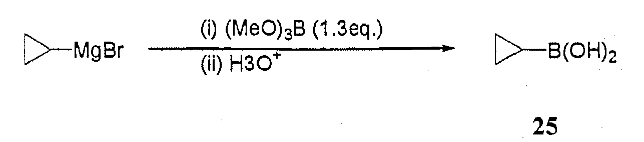

To a stirred solution of trimethylborate (1,69g, 1.81 ml, 16,25mmol) in THF (7ml) at -78°C under a N₂ atmosphere was added, by drop wise addition, cyclopropylmagnesium bromide (0,5M in THF, 25ml, 12.5mmol). A white precipitate formed. After 1 hr the reaction was warmed to room temperature and stirred overnight. The reaction was quenched with HCl aq. (20ml, 2.0N) and the mixture stirred for 1 hour. After extracting with DCM (15ml) and back extracting with H₂O (2x15ml), the aqueous fractions were combined and extracted using TBME (4x40ml). The combined organic extracts were dried over MgSO₄ and concentrated under reduced pressure to give a white solid. Recrystallisation from DCM and hexane (twice) furnished a white solid **25**, 297mg, 27% yield.
¹H nmr (250MHz, CDCl₃); 0.56-0.50 (2H, m, CH₂), 0,42-0.40 (2H, m, CH₂), -0.08-0.20 (1H, m, CH).

### 9-Cyclopropyl-5-methyl-1-phenyl-1,3,4,6-tetrahydro-5H-benz[f]-2,5-oxazocine (26a)

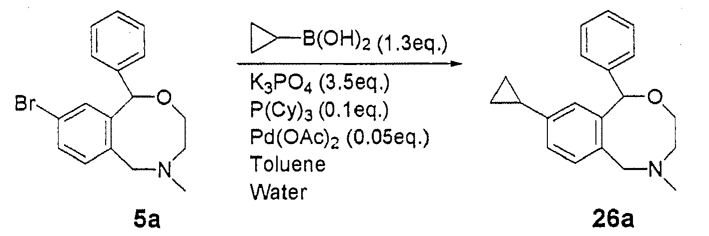

To a stirred solution of bromo-nefopam analogue **5a** (211 mg, 0.64mmol), cyclopropyl boronic acid (71 mg, 0.83mmol), potassium phosphate (472mg, 2.22mmol) and tricyclohexylphosphine (18mg, 0.06mmol) in toluene (5ml) and water (250 µl) under a N₂ atmosphere was added palladium acetate (7mg, 0.03mmol). The mixture was heated to 100°C for 3hrs and then cooled to room temperature. Water (10ml) was added and the mixture extracted with ethyl acetate (2x15ml). The combined organic extracts were washed with brine (10ml), dried over MgSO₄ and concentrated under reduced pressure to give a yellow oil. The crude product was purified by dry flash chromatography eluting with 15% MeOH in DCM. Fractions containing the product were combined and evaporated under reduced pressure to produce **26a** as a yellow oil. Yield 145mg, 78%.
¹H nmr (250MHz, CDCl₃); 7.32-7.24 (5H, m, CHₐᵣ), 7.11 (1H, d, *J* 7.8, CHₐᵣ), 6.87 (1H, dd, *J* 1.8, 7.8, CHₐᵣ), 6.73 (1H, d, *J* 1.5, CHₐᵣ), 5.74 (1H, s, CHO), 4.75 (1H, d, *J* 12.8, ArCHₐH_{b}), 4.17 (1H, ddd, *J* 2.8, 8.3, 12.4, OCHₐH_{b}), 3.85 (1H, ddd, *J* 2.3, 6.0, 12.5, OCHₐH_{b}), 3.63 (1H, d, *J* 12.8, ArCHₐH_{b}), 2.81 (1H, ddd, *J* 2.2, 8.2, 14.1, NCHₐH_{b}), 2.61 (1H, ddd, *J* 2.7, 5,9, 14.2, NCHₐH_{b}), 2.44 (3H, s, NCH₃), 1.77 (1H, m, CH), 0.91-0.86 (2H, m, CH₂), 0.61-0.57 (2H, m, CH₂),

### 8-Cyclopropyl-5-methyl-1-phenyl-1,3,4,6-tetrahydro-5H-benz[f]-2,5-oxazocine (26b)

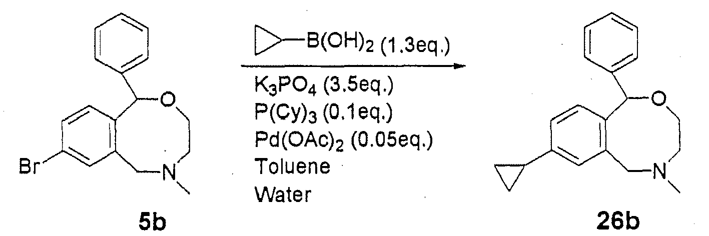

To a stirred solution of bromo-nefopam analogue **5b** (178mg, 0.54mmol), cyclopropyl boronic acid (60mg, 0.70mmol), potassium phosphate (398mg, 1.88mmol) and tricyclohexylphosphine (15mg, 0.05mmol) in toluene (4ml) and water (200 µl) under a N₂ atmosphere was added palladium acetate (6mg, 0.03mmol), The mixture was heated to 100°C for 3hrs and then cooled to room temperature. Water (10ml) was added and the mixture extracted with ethyl acetate (2x15ml). The combined organic extracts were washed with brine (10ml), dried over MgSO₄ and concentrated under reduced pressure to give a yellow oil. The crude product was purified by dry flash chromatography eluting with 15% MeOH in DCM. Fractions containing the product were combined and evaporated under reduced pressure to produce **26b** as a yellow oil. Yield 132mg, 84%.
¹H nmr (250MHz, CDCl₃); 7.31-7.23 (5H, m, CHₐᵣ), 6.94 (1H, s, CHₐᵣ), 6.86 (2H, d, *J* 1.0, CHₐᵣ), 5.78 (1H, s, CHO), 4.70 (1H, d, *J* 12.5, ArCHₐH_{b}), 4.15 (1H, ddd, *J* 2.6, 8.2, 12.6, OCHₐH_{b}), 3.86 (1H, ddd, *J* 2.1, 6.0, 12.7, OCHₐH_{b}), 3.66 (1H, d, *J* 12.7, ArCHₐH_{b}), 2.85 (1H, ddd, *J* 2.1, 8.2, 14.2, NCHₐH_{b}), 2.63 (1H, ddd, *J* 2.6, 6.0, 14.2, NCHₐH_{b}), 2.48 (3H, s, NCH₃), 1.86 (1H, m, CH), 0.96-0.90 (2H, m, CH₂), 0.72-0.66 (2H, m, CH₂).

### N-(Acetyl)-5-methyl-1-phenyl-1,3,4,6-tetrahydro-5H-benz[f]-2,5-oxazocine-9-methylamine (27a)

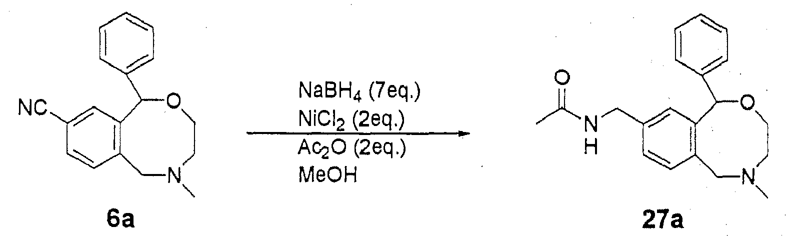

NaBH₄ (129mg, 3.41 mmol) was cautiously added to a solution of NiCl₂ (126mg, 0.97mmol), acetic anhydride (0.1 ml, 0.97mmol) and bromo-nefopam analogue 6a (134mg, 0.49mmol) in MeOH (8ml) at room temperature under a N₂ atmosphere. Once the vigorous reaction had subsided the mixture was left to stir at room temperature overnight. Methanol was removed under reduced pressure and the precipitate partially dissolved in EtOAc and NaHCO₃ (aq.). After filtration the green solid was repeatedly washed with EtOAc and NaHCO₃ (aq.). The organic filtrate was separated from the aqueous which was subsequently extracted with more EtOAc (3x10ml). The combined organic fractions were dried over MgSO₄, filtered and concentrated under reduced pressure. The crude product was purified by dry flash chromatography eluting with 20% MeOH in DCM. Fractions containing the product were combined and evaporated under reduced pressure to produce **27a** as an orange oil. Yield 36mg, 23%.
¹H nmr (250MHz, CDCl₃); 7.33-7.10 (7H, m, CHₐᵣ), 6.87 (1H, s, CHₐᵣ), 5.91 (1H, brs, NH), 5.73 (1H, s, CHO), 4.80 (1H, d, *J* 12.7, ArCHₐH_{b}), 4.29 (1H, d, *J* 2.9, NHCHₐCH_{b}), 4,27 (1H, d, *J* 2.9, NHCHₐCH_{b}), 4.17 (1H, m, OCHₐH_{b}), 3.82 (1H, ddd, *J* 2.1, 5.6, 12.6, OCHₐH_{b}), 3.65 (1H, d, *J* 12,7, ArCHₐH_{b}), 2.78 (1H, ddd, *J* 2.1, 8,4, 14.1, NCHₐH_{b}), 2.59 (1H, ddd, J2.5, 5.6, 14.1, NCHₐH_{b}), 2.43 (3H, s, NCH₃), 1.93 (3H, s, CH₃CO).

### N-(Acetyl)-5-methyl-1-phenyl-1,3,4,6-tetrahydro-5H-benz[f]-2,5-oxazocine-8-methylamine (27b)

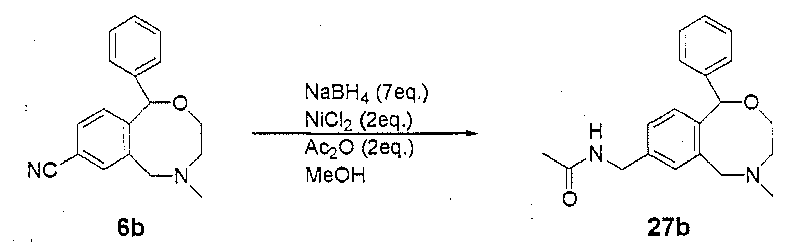

NaBH₄ (148mg, 3.92mmol) was cautiously added to a solution of NiCl₂ (145mg, 1.12mmol), acetic anhydride (0.11ml, 1.12mmol) and bromo-nefopam analogue **6b** (154mg, 0.56mmol) in MeOH (8ml) at room temperature under a N₂ atmosphere. Once the vigorous reaction had subsided the mixture was left to stir at room temperature overnight. Methanol was removed under reduced pressure and the precipitate partially dissolved in EtOAc and NaHCO₃ (aq.). After filtration the green solid was repeatedly washed with EtOAc and NaHCO₃ (aq.). The organic filtrate was separated from the aqueous which was subsequently extracted with more EtOAc (3x10ml). The combined organic fractions were dried over MgSO₄, filtered and concentrated under reduced pressure. The crude product was purified by dry flash chromatography eluting with 10-15% MeOH in DCM. Fractions containing the product were combined and evaporated under reduced pressure to produce **27b** as an orange oil. Yield 83mg, 46%.
¹H nmr (250MHz, CDCl₃); 7.30-7.23 (5H, m, CHₐᵣ), 7.14 (1H, s, CHₐᵣ), 7.11 (1H, d, *J* 8.1, CHₐᵣ), 6.97 (1H, d, *J* 7.8, CHₐᵣ), 5.83 (1H, brs, NH), 5.77 (1H, s, CHO), 4.75 (1H, d, *J* 12.5, ArCHₐH_{b}), 4.39 (2H, d, *J* 5.7, NHCH₂), 4.21-4.16 (1H, m, OCHₐH_{b}), 3.83 (1H, ddd, *J* 2.1, 6.2, 12.6, OCHₐH_{b}), 3.64 (1H, d, *J* 12.7, ArCHₐH_{b}), 2.84 (1H, ddd, *J* 2.1, 7.9, 14.2, NCHₐH_{b}), 2.62 (1H, ddd, *J* 2.6, 6.1, 14.2, NCHₐH_{b}), 2.49 (3H, s, NCH₃), 2.00 (3H, s, CH₃CO).

### N-(Methylsulphonyl)-5-methyl-1-phenyl-1,3,4,6-tetrahydro-5H-benz[f]-2,5-oxazocine-9-methylamine (28b)

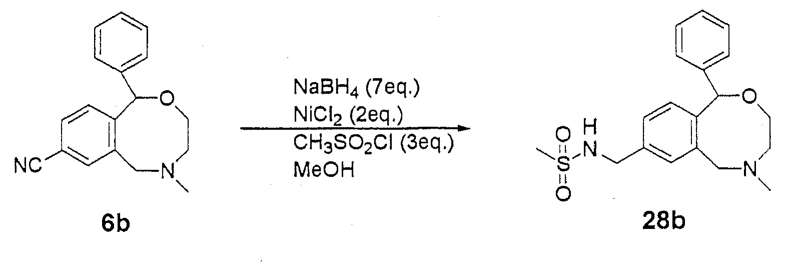

NaBH₄ (193mg, 5.11mmol) was cautiously added to a solution of NiCl₂ (188mg, 1.45mmol), methanesulfonyl chloride (0.17ml, 2.19mmol) and bromo-nefopam analogue **6b** (200mg, 0.73mmol) in MeOH (8ml) at room temperature under a N₂ atmosphere. Once the vigorous reaction had subsided the mixture was left to stir at room temperature overnight. Methanol was removed under reduced pressure and the precipitate partially dissolved in EtOAc and NaHCO₃ (aq.). After filtration the green solid was repeatedly washed with EtOAc and NaHCO₃ (aq.). The organic filtrate was separated from the aqueous which was subsequently extracted with more EtOAc (3x10ml). The combined organic fractions were dried over MgSO₄, filtered and concentrated under reduced pressure. The crude product was purified by dry flash chromatography eluting with 15-20% MeOH in DCM. Fractions containing the product were combined and evaporated under reduced pressure to produce **28b** as a white solid. Yield 54mg, 21 %.
¹H nmr (250MHz, CDCl₃); 7.95 (1H, s, CHₐᵣ), 7.77 (1H, dd, *J* 1.5, 8.1, CHₐᵣ), 7.39 (1H, d, *J* 8.2, CHₐᵣ), 7.33-7.24 (5H, m, CHₐᵣ), 6.00 (1H, s, CHO), 5.99 (1H, d, *J* 12.7, ArCHₐH_{b}), 4.64 (1H, m, OCHₐH_{b}), 4.48 (1H, d, *J* 12.8, ArCHₐH_{b}), 4.15 (1H, m, OCHₐH_{b}), 3.47 (3H, s, CH₃SO₂), 3.44 (1H, m, NCHₐH_{b}), 3.31 (1H, m, NCHₐH_{b}), 3.08 (2H, s, NHCH₂), 2.70 (3H, s, NCH₃).

### 9-Cyclopropyl-5-methyl-1-(3-methoxy)phenyl-1,3,4,6-tetrahydro-5H-benz[f]-2,5-oxazocine (29a)

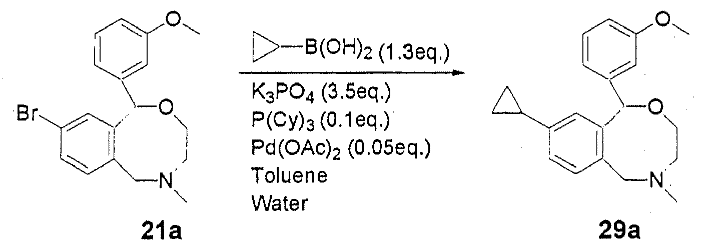

To a stirred solution of bromo-nefopam analogue **21a** (1 04mg, 0.29mmol), cyclopropyl boronic acid (32mg, 0.37mmol), potassium phosphate (214mg, 1.0mmol) and tricyclohexylphosphine (8mg, 0.03mmol) in toluene (5ml) and water (250 µl) under a N₂ atmosphere was added palladium acetate (3mg, 0.01 mmol). The mixture was heated to 100°C for 3hrs and then cooled to room temperature. Water (10ml) was added and the mixture extracted with ethyl acetate (2x15ml). The combined organic extracts were washed with brine (10ml), dried over MgSO₄ and concentrated under reduced pressure to give a yellow oil. The crude product was purified by dry flash chromatography eluting with 5% MeOH in DCM. Fractions containing the product were combined and evaporated under reduced pressure to produce **29a** as an orange oil. Yield 65mg, 70%.
¹H nmr (250MHz, CDCl₃); 7.23 (1H, t, *J* 8.1, CHₐᵣ), 7.10 (1H, d, *J* 7.8, CHₐᵣ), 6.88-6.75 (5H, m, CHₐᵣ), 5.70 (1H, s, CHO), 4.75 (1H, d, *J* 12.7, ArCHₐH_{b}), 4.21-4.12 (1H, m, OCHₐH_{b}), 3.84 (1H, ddd, *J* 2.3, 5.7, 12.6, OCHₐH_{b}), 3.77 (3H, s, OCH₃), 3.65 (1H, d, *J* 12.7, ArCHₐH_{b}), 2.82 (1H, ddd, *J* 2.2, 8.3, 14.1, NCHₐH_{b}), 2.62 (1H, ddd, *J* 2.7, 5,7, 14.2, NCHₐH_{b}), 2.45 (3H, s, NCH₃), 1.77 (1H, m, CH), 0.91-0.87 (2H, m, CH₂), 0.62-0.57 (2H, m, CH₂).

### 8-Cyclopropyl-5-methyl-1-(3-methoxy)phenyl-1,3,4,6-tetrahydro-5H-benz[f]-2,5-oxazocine (29b)

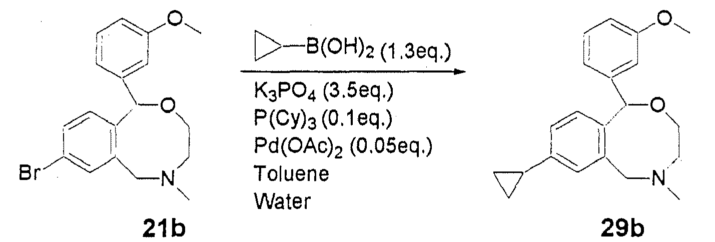

To a stirred solution of bromo-nefopam analogue 21 b (115mg, 0.32mmol), cyclopropyl boronic acid (36mg, 0.42mmol), potassium phosphate (241 mg, 1.13mol) and tricyclohexylphosphine (9mg, 0.03mmol) in toluene (4ml) and water (250 µl) under a N₂ atmosphere was added palladium acetate (4mg, 0,02mmol). The mixture was heated to 100°C for 3hrs and then cooled to room temperature. Water (10ml) was added and the mixture extracted with ethyl acetate (2x15ml). The combined organic extracts were washed with brine (10ml), dried over MgSO₄ and concentrated under reduced pressure to give a yellow oil. The crude product was purified by dry flash chromatography eluting with 5% MeOH in DCM. Fractions containing the product were combined and evaporated under reduced pressure to produce 29b as an orange oil. Yield 77mg, 75%.
¹H nmr (250MHz, CDCl₃); 7.22 (1H, t, *J* 8.1, CHₐᵣ), 6.94-6.76 (6H, m, CHₐᵣ), 5.74 (1H, s, CHO), 4.69 (1H, d, *J* 12.7, ACCHₐH_{b}); 4.14 (1H, ddd, *J* 2,6, 8.4, 12.6, OCHₐH_{b}), 3.85 (1H, ddd, *J* 2.1, 5.8, 12.7, OCHₐH_{b}), 3.77 (3H, s, OCH₃), 3.68 (1H, d, *J* 12.7, ArCHₐH_{b}), 2.85 (1H, ddd, *J* 2,0, 8.3, 14.2, NCHₐH_{b}), 2.64 (1H, ddd, *J* 2.5, 5.8, 14.3, NCHₐH_{B}) 2.49 (3H, s, NCH₃), 1.86 (1H, m, CH), 0.93 (2H, m, CH₂), 0.68 (2H, m, CH₂).

### 9-Cyclopropyl-5-methyl-1 -(4-methoxy)phenyl-1,3,4,6-tetrahydro-5H-benz[f]-2,6-oxazocine (30a)

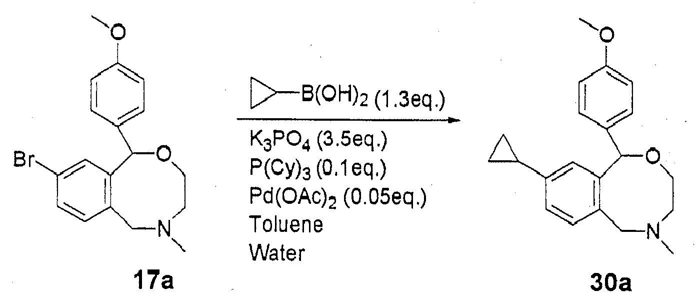

To a stirred solution of bromo-nefopam analogue **17a** (110mg, 0.31mmol), cyclopropyl boronic acid (34mg, 0.40mmol), potassium phosphate (226mg, 1.07mmol) and tricyclohexylphosphine (9mg, 0.03mmol) in toluene (4ml) and water (200 µl) under a N₂ atmosphere was added palladium acetate (4mg, 0.02mmol). The mixture was heated to 100°C for 3hrs and then cooled to room temperature. Water (10ml) was added and the mixture extracted with ethyl acetate (2x15ml). The combined organic extracts were washed with brine (10ml), dried over MgSO₄ and concentrated under reduced pressure to give a yellow oil. The crude product was purified by dry flash chromatography eluting with 5% MeOH in DCM. Fractions containing the product were combined and evaporated under reduced pressure to produce 30a as an orange oil. Yield 70mg, 71 %.
¹H nmr (250MHz, CDCl₃); 7.18 (2H, d, *J* 8.5, CHₐᵣ), 7.12 (1H, d, *J* 7.8, CHₐᵣ), 6.88 (1H, d, *J* 1.4, CHₐᵣ), 6.84 (2H, d, *J* 8.7, CHₐᵣ), 6.73 (1H, d, *J* 1.2, CHₐᵣ), 5.68 (1H, s, CHO), 4.80 (1H, d, *J* 12.7, ArCHₐH_{b}), 4.18 (1H, m, OCHₐH_{b}), 3.83 (1H, m, OCHₐH_{b}), 3.77 (3H, s, OCH₃), 3.64 (1H, d, *J* 12.7, ArCHₐH_{b}), 2.81 (1H, ddd, *J* 2.2, 8.5, 14.0, NCHₐH_{b}), 2.60 (1H, ddd, *J* 2.8, 5.5, 14.0, NCHₐH_{b}), 2.46 (3H, s, NCH₃), 1.77 (1H, m, CH), 0.87 (2H, m, CH₂), 0.60 (2H, m, CH₂).

### 8-Cyclopropyl-5-methyl-1 -(4-methoxy)phenyl-1,3,4,6-tetrahydro-5H-benz[f]-2,5-oxazocine (30b)

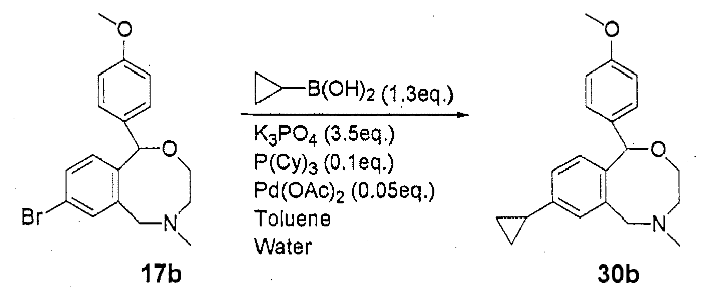

To a stirred solution of bromo-nefopam analogue 17b (117mg, 0.32mmol), cyclopropyl boronic acid (36mg, 0.42mmol), potassium phosphate (241 mg, 1.13mmol) and tricyclohexylphosphine (9mg, 0.03mmol) in toluene (4ml) and water (200 µl) under a N₂ atmosphere was added palladium acetate (4mg, 0.02mmol). The mixture was heated to 100°C for 3hrs and then cooled to room temperature. Water (10ml) was added and the mixture extracted with ethyl acetate (2x15ml). The combined organic extracts were washed with brine (10ml), dried over MgSO₄ and concentrated under reduced pressure to give a yellow oil. The crude product was purified by dry flash chromatography eluting with 5% MeOH in DCM. Fractions containing the product were combined and evaporated under reduced pressure to produce **30b** as an orange oil. Yield 62mg, 60%.
¹H nmr (250MHz, CDCl₃); 7.17 (2H, d, *J* 8.5, CHₐᵣ), 6.95 (1H, s, CHₐᵣ), 6.84 (4H, m, CHₐᵣ), 5.71 (1H, s, CHO), 4.77 (1H, d, *J* 12.5, ArCHₐH_{b}), 4.17 (1H, m, OCHₐH_{b}), 3.84 (1H, ddd, *J* 2.3, 5.5, 12.6, OCHₐH_{b}), 3.76 (3H, s, OCH₃), 3.66 (1H, d, *J* 12.5, ArCHₐH_{b}), 2,84 (1H, ddd, *J* 2.1, 8.5, 14.0, NCHₐH_{b}), 2.61 (1H, ddd, *J* 2.7, 5.5, 14.1, NCHₐH_{b}), 2.49 (3H, s, NCH₃), 1.85 (1H, m, CH), 0.93 (2H, m, CH₂), 0.67 (2H, m, CH₂).

### 9-Methoxy-5-methyl-1-(4-methoxy)phenyl-1,3,4,6-tetrahydro-5H-benz[f]-2,5-oxazocine (31a)

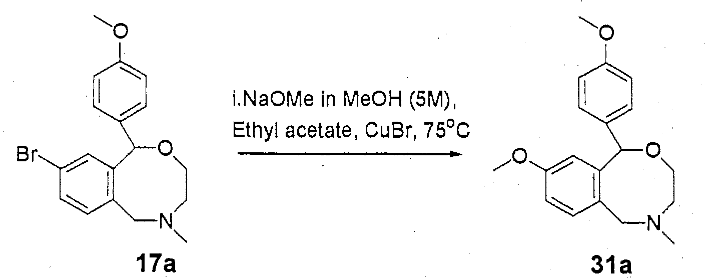

Ethyl acetate (0.1ml) was added to a stirred 5M NaOMe methanol solution (1ml) under an N₂ atmosphere at room temperature. Bromo-nefopam analogue **17a** (1 04mg, 0.29mmol) in MeOH (1ml) was then added followed by CuBr (8mg, 0.06mmol). The mixture was stirred at 75°C overnight, cooled to room temperature and quenched using water (5ml). The organic layer was separated and the aqueous was washed with ethyl acetate (2x10ml). The combined organics were dried over MgSO₄, filtered and concentrated under reduced pressure. The crude product was purified by dry flash chromatography eluting with 10% MeOH in DCM. Fractions containing the product were combined and evaporated under reduced pressure to produce **31a** as an orange oil. Yield 67mg, 71%.
¹H nmr (250MHz, CDCl₃); 7.18 (2H, d, *J* 8.5, CHₐᵣ), 7.19 (1H, s, CHₐᵣ), 6.83 (2H, d, *J* 8.6, CHₐᵣ), 6.77 (1H, dd, *J 2.5, 8.5,* CHₐᵣ), 6.52 (1H, d, *J* 2.3, CHₐᵣ), 5.72 (1H, s, CHO), 4.80 (1H, d, *J* 12.8, ArCHₐH_{b}), 4.20 (1H, m, OCHₐH_{b}), 3.84 (1H, ddd, *J* 2.4, 5.1, 12.8, OCHₐH_{b}), 3.77 (3H, s, OCH₃), 3.71 (3H, s, OCH₃), 3.68 (1H, d, *J* 12.8, ArCHₐH_{b}), 2.85 (1H, m, NCHₐH_{b}), 2.61 (1H, m, NCHₐH_{b}); 2.47 (3H, s, NCH₃).

### 8-Methoxy-5-methyl-1-(4-methoxy)phenyl-1,3,4,6-tetrahydro-5H-benz[f]-2,5-oxazocine (31 b)

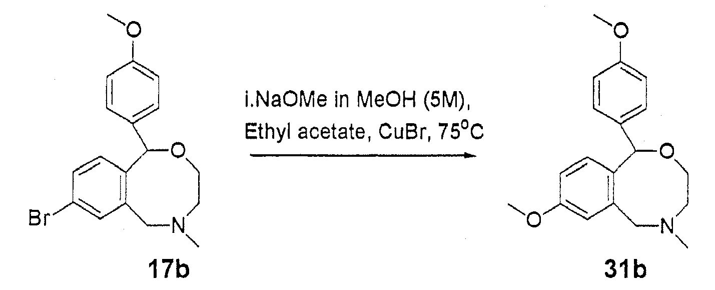

Ethyl acetate (0,1 ml) was added to a stirred 5M NaOMe methanol solution (1 ml) under an N₂ atmosphere at room temperature. Bromo-nefopam analogue **17b** (115mg, 0.32mmol) in MeOH (1 ml) was then added followed by CuBr (9mg, 0.06mmol). The mixture was stirred at 75°C overnight, cooled to room temperature and quenched using water (5ml). The organic layer was separated and the aqueous was washed with ethyl acetate (2x10ml). The combined organics were dried over MgSO₄, filtered and concentrated under reduced pressure, The crude product was purified by dry flash chromatography eluting with 10% MeOH in DCM, Fractions containing the product were combined and evaporated under reduced pressure to produce **31b** as an orange oil. Yield 39mg, 37%,
¹H nmr (250MHz, CDCl₃); 7.18 (2H, d, *J* 8.6, CHₐᵣ), 6.91 (1H, s, CHₐᵣ), 6.84 (2H, d, *J* 8.5, CHₐᵣ), 6.76 (1H, s, CHₐᵣ), 6.73 (1H, d, *J* 2.6, CHₐᵣ), 5.73 (1H, s, CHO), 4.71 (1H, d, *J* 12.7, ArCHₐH_{b}), 4.13 (1H, m, OCHₐH_{b}), 3.86 (1H, m, OCHₐH_{b}), 3.80 (3H, s, OCH₃), 3.77 (3H, s, OCH₃), 3.68 (1H, d, *J* 12.7, ArCHₐH_{b}), 2.86 (1H, m, NCHₐH_{b}), 2.64 (1H, ddd, *J* 2,5, 5.7, 14.1 NCHₐH_{b}), 2.48 (3H, s, NCH₃).

### 9-Cyano-5-methyl-1-(4-methoxy)phenyl-1,3,4,6-tetrahydro-5H-benz[f]-2,5-oxazocine (32a)

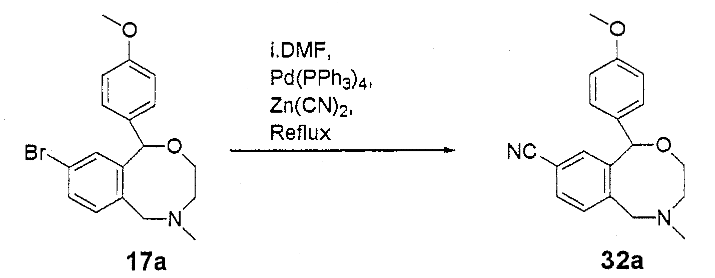

Bromo-nefopam analogue **17a** (133mg, 0.37mmol), Zn(CN)₂ (65mg, 0.55mmol), and Pd(PPh₃)₄ (63mg, 0.06mmol), were dissolved in degassed anhydrous DMF (4mL) under a N₂ atmosphere. The mixture was refluxed under N₂ for 24 hours. The mixture was allowed to cool to room temperature, filtered through celite and washed through with DCM (50ml), The filtrate was then quenched with water (10ml) and solvent extracted. The organic extract was dried over MgSO₄, filtered and solvent removed under reduced pressure. The crude product was purified by dry flash chromatography eluting with 5%-15% MeOH in DCM. Fractions containing the product were combined and evaporated under reduced pressure to produce **32a** as pale brown oil. Yield 32mg, 26%.
¹H nmr (250MHz, CDCl₃); 7.50 (1H, d, *J* 7.6, CHₐᵣ), 7.31 (2H, d, *J* 9.0, CHₐᵣ), 7.15 (2H, d, *J* 8.2, CHₐᵣ), 6.86 (2H, d, *J* 8.2, CHₐᵣ), 5.73 (1H, s, CHO), 4.91 (1H, d, *J* 12.8, ArCHₐH_{b}), 4.20 (1H, m, OCHₐH_{b}), 3.82 (4H, brm, OCHₐH_{b}, OCH₃), 3.68 (1H, d, *J* 12.8, ArCHₐH_{b}), 2.76 (1H, m, NCHₐH_{b}), 2.59 (1H, m, NCHₐH_{b}), 2.44 (3H, s, NCH₃).

### Biological Assays:

The assay was carried out according to the method described in, PEROVIC, S. and MULLER, W.E.G. (1995), Pharmacological profile of hypericum extract: effect on serotonin uptake by postsynaptic receptors, Arzneim-Forsch. Drug Res., 45: 1145.

### Assay for Inhibition of Noradrenaline Reuptake Activity:

The synaptosomes (100 µg) are incubated for 20 min at 37°C with 0.1 µCi [³H]norepinephrine in the absence (control) or presence of the test compound or the reference compound in a buffer containing 118 mM NaCl, 5 mM KCl, 2.5 mM MgSO₄, 1.2 mM NaH₂PO₄, 25 mM NaHCO₃, 11 mM glucose, 10 µM EGTA and 50 µM ascorbic acid (pH 7.4). Basal control activity is determined by incubating the same mixture for 20 min at 0°C in the presence of 10 µM protriptyline to block the uptake. Following incubation, the samples are filtered rapidly under vacuum through glass fiber filters (GF/B, Packard) and rinsed twice with ice-cold incubation buffer using a 96-sample cell harvester (Unifilter, Packard) to eliminate free [³H]norepinephrine, The filters are dried and the retained radioactivity is measured in a scintillation counter (Topcount, Packard) using a scintillation cocktail (Microscint 0, Packard).

The results are expressed as a percent inhibition of the control uptake of [³H]norepinephrine. The standard inhibitory reference compound is protriptyline, which is tested in each experiment at several concentrations to obtain an inhibition curve from which its IC₅₀ value is calculated.

### Assay for Inhibition of Serotonin Reuptake Activity:

The synaptosomes (100 µg) are incubated for 15 min at 37°C with 0.1 µCi [³H]serotonin in the absence (control) or presence of the test compound or the reference compound in a buffer containing 118 mM NaCl, 5 mM KCI, 2.5 mM MgSO₄, 1.2 mM NaH₂PO₄, 25 mM NaHCO₃, 11 mM glucose, 10 µM EGTA and 50 µM ascorbic acid (pH 7.4). Basal control activity is determined by incubating the same mixture for 15 min at 4°C in the presence of 10 µM imipramine to block the uptake. Following incubation, the samples are filtered rapidly under vacuum through glass fiber filters (GF/B, Packard) and rinsed twice with ice-cold incubation buffer using a 96-sample cell harvester (Unifilter, Packard) to eliminate free [³H]serotonin. The filters are dried and the retained radioactivity is measured in a scintillation counter (Topcount, Packard) using a scintillation cocktail (Microscint 0, Packard).

The results are expressed as a percent inhibition of the control uptake of [³H]serotonin, The standard inhibitory reference compound is imipramine, which is tested in each experiment at several concentrations to obtain an inhibition curve from which its IC₅₀ value is calculated.

## Claims

1. A compound of the general formula (1): 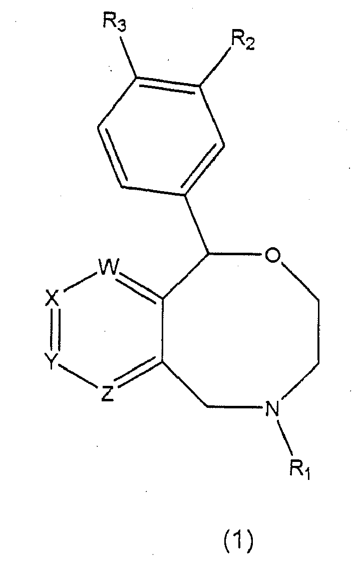 wherein
R₁ is H, C₁-C₆alkyl optionally substituted with F, C₃-C₆ cycloalkyl or C₂-C₆ alkenyl;
either R₂ and R₃ are the same or different and are H, halogen, CN, CF₃, C₁-C₆alkyl or OR₁; or R₂ and R₃ form a five or six membered ring which may be carbocyclic, heterocyclic (containing 1-2 heteroatoms selected from O, N and S), aromatic or heteroaromatic (containing 1-2 heteroatoms selected from O and N);
one of W, X, Y and Z is N or CR₄ and the others are each CH;
R₄ is a halogen atom, CF₃, CN, OR₇, SO₂N(R₆)₂, COR₆, CO₂R₆, CON(R₆)₂, NR₁COR₅, NR₁SO₂R₅, NR₁CO₂R_{5,} NR₁CON(R₆)₂, OC₁-C₆ alkyl optionally substituted with R₄, C₁-C₆ alkyl optionally substituted with R₄, C₃-C₆ cycloalkyl optionally substituted with R₄, C₂-C₆ alkenyl optionally substituted with R₄, C₂-C₆ alkynyl optionally substituted with R₄, aryl optionally substituted with R₄, or a five or six membered aromatic heterocycle containing 1-4 heteroatoms selected from N and O, linked either through carbon or nitrogen;
R₅ is C₁-C₆alkyl, C₂-C₆alkenyl, C₂-C₆ alkynyl, C₃-C₆cycloalkyl, aryl or heteroaryl;
each R₆ (which may be the same or different) is H, C₁-C₆ alkyl, C₂-C₆ alkenyl, C₂-C₆ alkynyl, C₃-C₆ cycloalkyl, aryl or heteroaryl; and
R₇ is aryl or heteroaryl;
or a pharmaceutically acceptable salt thereof.

2. A compound according to claim 1 which is exemplified herein.

3. A compound according to claim 1 or claim 2, for the treatment or prevention of a condition selected from depression, post-traumatic stress disorders, attention-deficit disorders, obsessive compulsive disorders, premenstural syndrome, substance abuse, micturition disorders and sexual dysfunction.

4. A compound according to claim 1 or claim 2, for the treatment or prevention of a condition which is acute, chronic or neuropathic pain, dysmennorhoea or migraine headache.

5. A compound according to claim 4, wherein the subject is also treated with an opiate.

6. A compound according to claim 4, wherein the subject is also treated with an analgesia inducer selected from acetaminophen, a non-steroidal anti-inflammatory drug, a narcotic analgesic, a local anesthetic, an NMDA antagonist, a neuroleptic agent, an anti-convulsant, an anti-spasmodic, an anti-depressant and a muscle relaxant.

7. A compound according to claim 1 or claim 2, for the treatment or prevention of emesis.

8. A compound according to claim 7, wherein the emesis is acute, delayed, post-operative, last-phase, or anticipatory emesis.

9. A compound according to claim 7, wherein the emesis is induced by chemotherapy, radiation, toxins, pregnancy, vestibular disorder, motion, post-operative sickness, surgery, gastrointestinal obstruction, reduced gastrointestinal motility, visceral pain, migraine or opioid analgesics.
